# EUROPEAN PATENT APPLICATION

(11) **EP 4 435 002 A1**
(43) Date of publication of application: **25.09.2024**
(21) Application number: 22897980.3
(22) Date of filing: 29.11.2022
(51) Int. Cl.: C07K 16/00, C07K 16/18, C07K 16/42, A61P 35/00

(54) **MODIFIED PROTEIN OR POLYPEPTIDE**

(30) Priority: 29.11.2021 CN 202111429892
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: WANG, Nan, Shanghai 201210 (CN); YANG, Xiaofeng, Shanghai 201210 (CN); ZHANG, Wei, Shanghai 201210 (CN); CAO, Zhiliang, Shanghai 201210 (CN); YE, Hao, Shanghai 201210 (CN); SHEN, Yuhong, Shanghai 201210 (CN); LIAO, Cheng, Shanghai 201210 (CN)
(74) Representative: Potter Clarkson
(86) International application number: PCT/CN2022/134923
(87) International publication number: WO 2023/093899

(57) **Abstract**

Provided are a modified immunoglobulin single variable domain, and a protein or polypeptide comprising same. The modified immunoglobulin single variable domain has reduced binding to a pre-anti-drug antibody. Specifically, the C terminal of the immunoglobulin single variable domain comprises amino acid modification. The present invention further relates to an immunoglobulin single variable domain, the pharmaceutical use of a protein or polypeptide comprising same, a pharmaceutical composition, an encoding nucleic acid, and a preparation method.

## Description

The present disclosure claims priority to the Chinese Patent Application (Application No. CN202111429892.3) filed on Nov. 29, 2021.

### TECHNICAL FIELD

The present disclosure relates to the field of biopharmaceutics and particularly to a modified immunoglobulin single variable domain and a protein or polypeptide comprising the modified immunoglobulin single variable domain, which have reduced binding to pre-anti-drug antibodies. The C-terminus of the immunoglobulin single variable domain comprises an amino acid modification. The present disclosure further relates to pharmaceutical use of the immunoglobulin single variable domain or the protein or polypeptide comprising the immunoglobulin single variable domain, a pharmaceutical composition thereof, a coding nucleic acid therefor, and a preparation method therefor.

### BACKGROUND

Immunogenicity is common among antibody drugs. According to reports related to preclinical and clinical trials, various marketed or experimental antibody drugs can induce anti-drug antibodies (ADAs) in animals, and these ADAs may affect the properties such as the therapeutic effect and *in vivo* metabolism of the antibody drugs. Over the years, several techniques and methods have been developed to circumvent or ease this problem, including the preparation of chimeric antibodies, the humanization engineering of antibodies, the development of fully human antibodies, and the like.

However, even fully human antibodies cannot completely avoid immunogenicity. One of the reasons for this is the presence of autoantibodies in the human body that can bind to natural immunoglobulins or fragments of immunoglobulins, i.e., pre-anti-drug antibodies (pre-ADAs). These pre-ADAs are specific for epitopes of various immunoglobulins that exist throughout the human body, the epitopes including those in the Fc region, the variable regions/CDRs, the hinge region of the heavy chain, and the like. According to current research on their functions, these pre-ADAs are associated with infection and the homeostasis of the body's immune system, though many specific functions are not yet fully understood.
pre-ADAs can bind to therapeutic proteins or polypeptides (including monoclonal antibodies) or polypeptide molecules in addition to endogenous immunoglobulins. pre-ADAs that bind to therapeutic protein or polypeptide molecules can affect their function and metabolism by maintaining, eliminating, or neutralizing the molecules, resulting in changes in clinical response and bioavailability, and even side effects such as hypersensitivity and special events.

Single-domain antibodies (sdAbs), including those derived from the heavy chain variable regions (VHs) of human and non-human primate IgG molecules and those from the heavy chains (VHHs) of camelids/alpacas, generally undergo immunogenicity-related testing and optimization before entering clinical development to reduce ADAs. Despite humanization engineering, these molecules are still likely to bind to pre-ADAs. There have been reports of strong immune-related side effects in the clinical development of drug molecules based on the structures of sdAbs and their derivatives, which are closely related to the binding of pre-ADAs.

Therefore, providing engineering methods that can reduce the binding of sdAbs and their derivatives to pre-ADAs is highly beneficial for better developing corresponding drugs and maximizing their clinical value. In the present disclosure, we tested a series of engineering methods aimed at reducing the binding of sdAbs to pre-ADAs and succeeded in obtaining relatively ideal candidate molecules. These engineering methods and the resulting candidate molecules are expected to be widely used in the future to reduce the binding of sdAb-based therapeutic biomacromolecules to pre-ADAs, thereby optimizing the clinical therapeutic effects of drugs.

### SUMMARY

The present disclosure provides an immunoglobulin single variable domain having reduced binding to anti-drug antibodies (ADAs) and pre-anti-drug antibodies (pre-ADAs). In addition, the present disclosure provides the immunoglobulin single variable domain and a protein or polypeptide comprising the immunoglobulin single variable domain, and a coding nucleic acid for a related product, a preparation method therefor, a pharmaceutical composition thereof, pharmaceutical use thereof, and detection use thereof.

### Immunoglobulin Single Variable Domain

In a first aspect, some embodiments of the present disclosure provide an immunoglobulin single variable domain comprising:
a C-terminus the amino acid sequence of which is represented by X₁X₂(X₃)ₙ or VTVX₁X₂(X₃)ₙ, wherein X₁ is selected from the group consisting of S, F, and R, X₂ is selected from the group consisting of A, P, and S, and X₃ is A or absent; n is an integer of 0-5;
when n is 0, X₃ is absent;
when n is 1-5, X₃ is A.

In some specific embodiments, the modified immunoglobulin single variable domain comprises a C-terminus consisting of any one of the following (a)-(e):
(a) SAA or VTVSAA (SEQ ID NO: 37);
(b) FP or VTVFP (SEQ ID NO: 38);
(c) FPA or VTVFPA (SEQ ID NO: 39);
(d) RS or VTVRS (SEQ ID NO: 45); and
(e) RSA or VTVRSA (SEQ ID NO: 46).

In some embodiments, provided is an immunoglobulin single variable domain the C-terminus of which comprises or ends with an amino acid sequence represented by X₁X₂X₃ or VTVX₁X₂X₃, wherein X₁ is selected from the group consisting of S, F, and R; X₂ is selected from the group consisting of A, P, and S, or X₂ is absent; X₃ is selected from the group consisting of A, or X₃ is absent.

In some embodiments, the X₁X₂X₃ or VTVX₁X₂X₃ is selected from the group consisting of any one of the following (1)-(10):
(1) SAA or VTVSAA (SEQ ID NO: 37);
(2) FP or VTVFP (SEQ ID NO: 38);
(3) FPA or VTVFPA (SEQ ID NO: 39);
(4) RP or VTVRP (SEQ ID NO: 40);
(5) R or VTVR (SEQ ID NO: 41);
(6) RA or VTVRA (SEQ ID NO: 42);
(7) FS or VTVFS (SEQ ID NO: 43);
(8) SP or VTVSP (SEQ ID NO: 44);
(9) RS or VTVRS (SEQ ID NO: 45); and
(10) RSA or VTVRSA (SEQ ID NO: 46).

In some embodiments, provide is an immunoglobulin single variable domain the C-terminus of which comprises or ends with any one of the following amino acid sequences: SA, SAS, SAST (SEQ ID NO: 47), SASTK (SEQ ID NO: 48), SASTKG (SEQ ID NO: 49), SASTKGP (SEQ ID NO: 50), SAT, SATV (SEQ ID NO: 51), SAAS (SEQ ID NO: 52), SAAST (SEQ ID NO: 53), SAASTK (SEQ ID NO: 54), SAASTKG (SEQ ID NO: 55), SAASTKGP (SEQ ID NO: 56), FS, FSA, FSAS (SEQ ID NO: 57), FSAST (SEQ ID NO: 58), FSASTK (SEQ ID NO: 59), FSASTKG (SEQ ID NO: 60), FSASTKGP (SEQ ID NO: 61), FST, FSTV (SEQ ID NO: 62), FPAS (SEQ ID NO: 63), FPAST (SEQ ID NO: 64), FPASTK (SEQ ID NO: 65), FPASTKG (SEQ ID NO: 66), FPASTKGP (SEQ ID NO: 67), RPA, RPAA (SEQ ID NO: 68), RPAST (SEQ ID NO: 69), RPASTK (SEQ ID NO: 70), RPASTKG (SEQ ID NO: 71), RPASTKGP (SEQ ID NO: 72), RPT, RPTV (SEQ ID NO: 73), RPAAST (SEQ ID NO: 74), RPAASTK (SEQ ID NO: 75), RPAASTKG (SEQ ID NO: 76), RPAASTKGP (SEQ ID NO: 77), RAA, PAST (SEQ ID NO: 78), RASTK (SEQ ID NO: 79), RASTKG (SEQ ID NO: 80), RASTKGP (SEQ ID NO: 81), RAAST (SEQ ID NO: 82), RAASTK (SEQ ID NO: 83), RAASTKG (SEQ ID NO: 84), RAASTKGP (SEQ ID NO: 85), RAT, RATV (SEQ ID NO: 86), SAAG (SEQ ID NO: 87), SAAGG (SEQ ID NO: 88), FPG, FPGG (SEQ ID NO: 89), FPAG (SEQ ID NO: 90), FPAGG (SEQ ID NO: 91), RAG, RAGG (SEQ ID NO: 92), RS, RSA, RSAA (SEQ ID NO: 93), RSAAA (SEQ ID NO: 94), RSAS (SEQ ID NO: 95), RSAST (SEQ ID NO: 96), RSASTK (SEQ ID NO: 97), RSASTKG (SEQ ID NO: 98), RSASTKGP (SEQ ID NO: 99), RSG, or RSGG (SEQ ID NO: 100); VTVSA (SEQ ID NO: 101), VTVSAS (SEQ ID NO: 102), VTVSAST (SEQ ID NO: 103), VTVSASTK (SEQ ID NO: 104), VTVSASTKG (SEQ ID NO: 105), VTVSASTKGP (SEQ ID NO: 106), VTVSAT (SEQ ID NO: 107), VTVSATV (SEQ ID NO: 108), VTVSAAS (SEQ ID NO: 109), VTVSAAST (SEQ ID NO: 110), VTVSAASTK (SEQ ID NO: 111), VTVSAASTKG (SEQ ID NO: 112), VTVSAASTKGP (SEQ ID NO: 113), VTVSAT (SEQ ID NO: 114), VTVSATV (SEQ ID NO: 115), VTVFS (SEQ ID NO: 116), VTVFSA (SEQ ID NO: 117), VTVFSAS (SEQ ID NO: 118), VTVFSAST (SEQ ID NO: 119), VTVFSASTK (SEQ ID NO: 120), VTVFSASTKG (SEQ ID NO: 121), VTVFSASTKGP (SEQ ID NO: 122), VTVFST (SEQ ID NO: 123), VTVFSTV (SEQ ID NO: 124), VTVFPAS (SEQ ID NO: 125), VTVFPAST (SEQ ID NO: 126), VTVFPASTK (SEQ ID NO: 127), VTVFPASTKG (SEQ ID NO: 128), VTVFPASTKGP (SEQ ID NO: 129), VTVRPA (SEQ ID NO: 130), VTVRPAA (SEQ ID NO: 13 1), VTVRPAST (SEQ ID NO: 132), VTVRPASTK (SEQ ID NO: 133), VTVRPASTKG (SEQ ID NO: 134), VTVRPASTKGP (SEQ ID NO: 135), VTVRPAA (SEQ ID NO: 136), VTVRPT (SEQ ID NO: 137), VTVRPTV (SEQ ID NO: 138), VTVRPAAST (SEQ ID NO: 139), VTVRPAASTK (SEQ ID NO: 140), VTVRPAASTKG (SEQ ID NO: 141), VTVRPAASTKGP (SEQ ID NO: 142), VTVRAA (SEQ ID NO: 143), VTVRAST (SEQ ID NO: 144), VTVRASTK (SEQ ID NO: 145), VTVRASTKG (SEQ ID NO: 146), VTVRASTKGP (SEQ ID NO: 147), VTVRAAST (SEQ ID NO: 148), VTVRAASTK (SEQ ID NO: 149), VTVRAASTKG (SEQ ID NO: 150), VTVRAASTKGP (SEQ ID NO: 151), VTVRAT (SEQ ID NO: 152), VTVRATV (SEQ ID NO: 153), VTVSAAG (SEQ ID NO: 154), VTVSAAGG (SEQ ID NO: 155), VTVFPG (SEQ ID NO: 156), VTVFPGG (SEQ ID NO: 157), VTVFPAG (SEQ ID NO: 158), VTVFPAGG (SEQ ID NO: 159), VTVRAG (SEQ ID NO: 160), VTVRAGG (SEQ ID NO: 161), VTVRS (SEQ ID NO: 162), VTVRSA (SEQ ID NO: 163), VTVRSAA (SEQ ID NO: 164), VTVRSAAA (SEQ ID NO: 165), VTVRSAS (SEQ ID NO: 166), VTVRSAST (SEQ ID NO: 167), VTVRSASTK (SEQ ID NO: 168), VTVRSASTKG (SEQ ID NO: 169), VTVRSASTKGP (SEQ ID NO: 170), VTVRSG (SEQ ID NO: 171), or VTVRSGG (SEQ ID NO: 172).

In a second aspect, some embodiments of the present disclosure provide an immunoglobulin single variable domain the C-terminus of which comprises or ends with an amino acid sequence represented by X₄X₅ or VTVX₄X₅, wherein:
X₄ is selected from the group consisting of F or R, and X₅ may be present or absent; when X₅ is present, it represents an amino acid extension of 1-10 (e.g., 1, 2, 3, 4, 5, 6, 7, 8, 9, or 10, or e.g., 1-3, 1-4, 1-5, 1-6, 1-7, 1-8, or 1-9) amino acid residues; the amino acid extension is, e.g., P, PA, A, AA, AAA, AAAA (SEQ ID NO: 173), PAA, PAAA (SEQ ID NO: 174), AS, AST, ASTK (SEQ ID NO: 175), ASTKG (SEQ ID NO: 176), ASTKGP (SEQ ID NO: 177), PAS, PAST (SEQ ID NO: 178), PASTK (SEQ ID NO: 179), PASTKG (SEQ ID NO: 180), PASTKGP (SEQ ID NO: 181), PT, PTV, PG, PGG, PAG, S, SA, SAA, SAAA (SEQ ID NO: 182), SAS, SSAST (SEQ ID NO: 183), SSASTK (SEQ ID NO: 184), SSASTKG (SEQ ID NO: 185), SSASTKGP (SEQ ID NO: 186), SRSG (SEQ ID NO: 187), or SRSGG (SEQ ID NO: 188); or
X₄ is S, and X₅ represents an amino acid extension of 1-10 (e.g., 1, 2, 3, 4, 5, 6, 7, 8, 9, or 10, or e.g., 1-3, 1-4, 1-5, 1-6, 1-7, 1-8, or 1-9) amino acid residues; the amino acid extension is, e.g., A, AA, AAA, AAAA, AS, AST, ASTK, ASTKG, ASTKGP, T, TV, G, or GG.

In some embodiments, provided is an immunoglobulin single variable domain the C-terminus of which comprises or ends with an amino acid sequence represented by X₄X₅ or VTVX₄X₅, wherein:
X₄ is selected from the group consisting of F or R, and X₅ represents an amino acid extension of 1 to 5 amino acid residues; the amino acid extension is preferably P, PA, S, or SA; or
X₄ is S, and X₅ represents an amino acid extension of 1 to 5 amino acid residues; the amino acid extension is preferably AA;
for example, the X₄X₅ or VTVX₄X₅ is selected from the group consisting of any one of the following:
   SAA or VTVSAA;
   FP or VTVFP;
   FPA or VTVFPA;
   RS or VTVRS; or
   RSA or VTVRSA.

In a third aspect, some embodiments of the present disclosure provide an immunoglobulin single variable domain comprising a framework sequence derived from a human germline.

In some embodiments, the immunoglobulin single variable domain has an amino acid modification at position 112 relative to the human germline framework sequence, e.g., selected from the group consisting of 112F, 112R, or 112A.

In some embodiments, the immunoglobulin single variable domain has an amino acid modification at position 113 relative to the human germline framework sequence, e.g., 113P, 113A, or 113S.

In some embodiments, when an amino acid modification of 112F is present, an amino acid modification of 113P, 113A, or 113S is present at position 113; or, when an amino acid modification of 112R is present, and/or an amino acid modification of 113P or 113A is present at position 113.

In some embodiments, the immunoglobulin single variable domain has, at position 112 and position 113, an amino acid modification combination selected from the group consisting of any one of the following: 112F and 113P, 112F and 113A, 112F and 113S, 112R and 113P, 112R and 113A, 112R and 113S, 112A and 113P, 112A and 113A, 112A and 113S, and 112S and 113P.

In some embodiments, provided is an immunoglobulin single variable domain having an amino acid modification of 112F or 112R at position 112 relative to a human germline framework sequence;
optionally, the immunoglobulin single variable domain further has an amino acid modification of 113P at position 113 relative to the human germline framework sequence, or has an S at position 113.

In some specific embodiments, the immunoglobulin single variable domain has an amino acid residue combination of any one of the following at position 112 and position 113 relative to the human germline framework sequence:
112F and 113P;
112R and 113S; and
112F and 113S.

In a fourth aspect, some embodiments of the present disclosure provide an immunoglobulin single variable domain comprising a framework sequence derived from a human germline.

In some embodiments, the immunoglobulin single variable domain has an S at position 112 relative to a human germline heavy chain framework sequence and comprises an amino acid modification at position 113 relative to the human germline heavy chain framework sequence, the modification being selected from the group consisting of 113A and 113P.

In some embodiments, the immunoglobulin single variable domain also has an amino acid extension of 1-10 (e.g., 1, 2, 3, 4, 5, 6, 7, 8, 9, or 10, or e.g., 1-3, 1-4, 1-5, 1-6, 1-7, 1-8, or 1-9) amino acid residues after position 113; the amino acid extension is, e.g., A, AA, AAA, AAAA, AS, AST, ASTK, ASTKG, ASTKGP, TV, T, G, or GG.

In some embodiments, the aforementioned C-terminus may be produced via amino acid modifications (e.g., substitutions or replacements, deletions, or additions), so that the immunoglobulin single variable domain comprises the amino acid modifications.

In some embodiments, the aforementioned immunoglobulin single variable domain may further comprise:
1) one or more amino acid modifications at position 14, position 41, and position 108 relative to the human germline framework sequence. In some specific embodiments, modifications at position 14 include, but are not limited to, 14A, 14K, 14Q, and 14T (e.g., P 14A, P 14K, P14Q, and P14T); modifications at position 41 include, but are not limited to, 41A (e.g., P41A); and modifications at position 108 include, but are not limited to, 108A and 108Q (e.g., L108A and L108Q). The relevant mutations in WO2013024059A are incorporated herein by reference in their entirety.
2) one or more amino acid modifications at position 11, position 13, position 14, position 15, position 82, position 82a or position 82b, position 83, position 84, position 107, and position 108 relative to the human germline framework sequence. The relevant mutations in WO2012175741A are incorporated herein by reference in their entirety.
3) amino acid modifications at position 11 and/or position 88 relative to the human germline framework sequence. In some specific embodiments, modifications at position 11 include, but are not limited to, 11K, 11R, 11D, and 11E (e.g., L IlK, L11R, L11D, and L11E), and modifications at position 88 include, but are not limited to, 88E, 88D, 88R, and A8K (e.g., A88E, A88D, A88R, and A88K). In some specific embodiments, the immunoglobulin single variable domain has a combination selected from the group consisting of 11E/88E, 11E/88D, 11E/88K, 11E/88R, 11D/88E, 11D/88D, 11D/88K, 11D/88R, 11K/88E, 11K/88D, 11K/88K, 11K/88R, 11R/88E, 11R/88D, 11R/88K, or 11R/88R (e.g., L11E/A88E, L11E/A88D, L11E/A88K, L11E/A88R, L11D/A88E, L11D/A88D, L11D/A88K, L11D/A88R, L11K/A88E, L11K/A88D, L11K/A88K, L11K/A88R, L11R/A88E, L11R/A88D, L11R/A88K, or L11R/A88R). The relevant mutations in WO2016118733 are incorporated herein by reference in their entirety.

In some embodiments, the aforementioned human germline framework sequence may be a human germline heavy chain framework sequence.

In some embodiments, the aforementioned modified immunoglobulin single variable domain provided by the present disclosure has reduced side effects and/or increased safety, as compared to an unmodified immunoglobulin single variable domain. For example, the modified immunoglobulin single variable domain has reduced immunogenicity.

In some specific embodiments, the aforementioned modified immunoglobulin single variable domain provided by the present disclosure has reduced binding to pre-ADAs or ADAs, e.g., present in serum, as compared to an unmodified immunoglobulin single variable domain. Reduced binding capacity means that the molecule binds to pre-ADAs (or ADAs) with reduced affinity or reduced avidity. For example, the K_{D} between the aforementioned modified immunoglobulin single variable domain provided by the present disclosure and pre-ADAs (or ADAs) is 1.5 or more times (e.g., 2, 2.5, 3, 3.5, 4, 4.5, 5, 5.5, 6, 6.5, 7, or 8 times) the K_{D} between an unmodified sdAb and pre-ADAs (or ADAs). As another example, the binding of the aforementioned modified immunoglobulin single variable domain provided by the present disclosure to pre-ADAs (or ADAs) is reduced by 10% or more (e.g., 20%, 30%, 35%, 40%, 45%, 50%, 55%, 60%, 65%, 70%, 80%, or 90%), as compared to the binding of an unmodified sdAb to pre-ADAs (or ADAs); assay methods that may be used are conventional in the art, e.g., the ELISA shown in Example 1 of the present disclosure. K_{D} refers to the equilibrium dissociation constant, and those skilled in the art understand that the smaller the value of K_{D}, the stronger the binding.

In some embodiments, the aforementioned immunoglobulin single variable domain provided by the present disclosure may be a single-domain antibody (sdAb) or nanobody. For example, the immunoglobulin single variable domain is a VH sdAb or VHH. The immunoglobulin single variable domain may be of human or non-human origin, e.g., a human VH sdAb or a humanized camelid VHH. The immunoglobulin single variable domain (e.g., sdAb or nanobody) may be obtained by phage display, or may be chimeric, of camelid origin, or humanized. The immunoglobulin single variable domain (e.g., sdAb or nanobody) may have undergone sequence optimization, such as humanization, affinity maturation, removal of T cell epitopes, reduction of antibody deamidation, and/or sequence engineering for reducing antibody isomerization. The immunoglobulin single variable domain (e.g., sdAb) may be post-translationally modified, such as the cleavage of leader sequences, the addition of various sugar moieties in various glycosylation and phosphorylation patterns, deamidation, oxidation, disulfide bond scrambling, isomerization, C-terminal lysine clipping, and N-terminal glutamine cyclization.

In some embodiments, the aforementioned immunoglobulin single variable domain provided by the present disclosure binds (e.g., monovalently) to the target antigen, including specific binding to a CDR of the target antigen (e.g., including the CDR1, CDR2, and CDR3); the sdAb binds to the target antigen, e.g., with a K_{D} in the range of about 5 µM to about 1 pM, or e.g., with a K_{D} in the range of about 500 nM to about 10 pM, about 200 nM to about 10 pM, about 50 nM to about 10 pM, or about 10 nm to about 10 pM.

In some embodiments, the aforementioned immunoglobulin single variable domain provided by the present disclosure includes a variant thereof that has one or more (e.g., 1, 2, 3, 4, 5, 6, 7, 8, 9, or 10) amino acid mutations, as compared to the parent immunoglobulin single variable domain; the amino acid mutations may be conservative replacements, substitutions, or modifications, and/or deletions or additions that do not affect function; the amino acid mutations may occur in the CDRs and/or FRs; the variant has the same or highly similar biological function and binding affinity for pre-ADAs or ADAs to the parent immunoglobulin single variable domain. In some specific embodiments, the variant is, e.g., an amino acid conservative substitution variant of the immunoglobulin single variable domain.

In some embodiments, the aforementioned immunoglobulin single variable domain of the present disclosure is fused or conjugated to an additional molecule to be present in the form of a fusion or conjugate. The additional molecule includes, but is not limited to: additional immunoglobulin single variable domains, proteins or polypeptides, antibodies or antigen-binding fragments thereof, the Fc regions of immunoglobulins, and PEG molecules. In some specific embodiments, the aforementioned immunoglobulin single variable domain of the present disclosure is located or exposed at the C-terminus of the fusion or conjugate; for example, where the fusion or conjugate consists of one polypeptide chain, the immunoglobulin single variable domain of the present disclosure is located or exposed at the C-terminus of the polypeptide chain; where the fusion or conjugate is formed by the polymerization of two or more polypeptide chains, the immunoglobulin single variable domain of the present disclosure is located or exposed at the C-terminus of at least one of the polypeptide chains, or at the C-termini of two or more of the polypeptide chains.

### Protein or Polypeptide

The present disclosure provides a protein or polypeptide comprising at least one (e.g., 1, 2, 3, 4, 5, 6, 7, or 8) of the aforementioned immunoglobulin single variable domains of the present disclosure.

In some embodiments, the protein or polypeptide is a fusion protein or polypeptide of an immunoglobulin single variable domain of the present disclosure and another polypeptide (e.g., a therapeutic polypeptide).

In some embodiments, the at least one immunoglobulin single variable domain is located at the C-terminus of the protein or polypeptide.

In some embodiments, the protein or polypeptide is an antibody (including an antigen-binding fragment thereof).

In some embodiments, the antibody is a monospecific antibody (mAb) or a bispecific antibody (diabody), or a multispecific antibody (e.g., triabody or tetrabody).

In some embodiments, the antibody is monovalent, bivalent, trivalent, tetravalent, pentavalent, or hexavalent.

In some embodiments, the antibody comprises a heavy chain and a light chain, and the at least one immunoglobulin single variable domain is located at the C-terminus of the heavy chain and/or the C-terminus of the light chain.

In some embodiments, where the protein or polypeptide (e.g., an antibody) consists of one polypeptide chain, the immunoglobulin single variable domain of the present disclosure is located at the C-terminus of the polypeptide chain; where the protein or polypeptide (e.g., an antibody) consists of/is formed by the polymerization of two polypeptide chains, the immunoglobulin single variable domain of the present disclosure may be located at the C-terminus of one or both of the two polypeptide chains; where the protein or polypeptide (e.g., an antibody) consists of/is formed by the polymerization of three, four, or more polypeptide chains, the immunoglobulin single variable domain of the present disclosure may be located at the C-terminus of one, two, more, or all of the polypeptide chains. In the present disclosure, instances where another immunoglobulin variable domain, Fab, Fab', F(ab')2, Fv, Fab-Fv, Fab-dsFv, single-domain antibody (e.g., VH sdAb or VL sdAb or VHH), scFv, or polypeptide is linked to the N-terminus of the polypeptide chain are not excluded.

In some embodiments, the protein or polypeptide (e.g., an antibody) comprises an immunoglobulin Fc region; for example, the Fc region is the Fc region of human IgG1, IgG2, IgG3, or IgG4. In some specific embodiments, the Fc region has increased or decreased effector function, and the effector function is antibody-dependent cell-mediated cytotoxicity (ADCC), antibody-dependent cellular phagocytosis (ADCP), or complement-dependent cytotoxicity (CDC). In some specific embodiments, the Fc region has increased or decreased binding to FcyRs. In some specific embodiments, the Fc region has a heavy chain mismatch-preventing mutation, such as Knob-into-hole (KIH). In some specific embodiments, an exemplary IgG1 Fc region with enhanced effector function comprises and has the following substitutions or any combination thereof S239D, S239E, S239K, F241A, V262A, V264D, V264L, V264A, V264S, D265A, D265S, D265V, F296A, Y296A, R301A, I332E; e.g., S239D/I332E, S239D/A330S/I332E, S239D/A330L/I332E, S298A/D333A/K334A, P247I/A339D, P247I/A339Q, D280H/K290S, D280H/K290S/S298D, D280H/K290S/S298V, F243L/R292P/Y300L, F243L/R292P/Y300L/P396L, F243L/R292P/Y300L/V305I/P396L, G236A/S239D/I332E, K326A/E333A, K326W/E333S, K290E/S298G/T299A, K290N/S298G/T299A, K290E/S298G/T299A/K326E, or K290N/S298G/T299A/K326E. In some embodiments, exemplary IgG Fc regions with reduced effector function comprise and has the following substitutions: L234A/L235A, L234F/L235E/D265A, or L234A/L235A/G237A/P238S/H268A/A330S/P331S, K214T/E233P/L234V/L235A/G236 deletion/A327G/P331A/D365E/L358M on IgG1; V234A/G237A, V234A/G237A/P238S/H268A/V309L/A330S/P331S, H268Q/V309L/A330S/P331S, S267E/L328F on IgG2; F234A/L235A, S228P/F234A/L235A, S228P/F234A/L235A/G236 deletion/G237A/P238S, S228P/F234A/L235A/G237A/P238S on IgG4; N297A on IgG1, IgG2, IgG3, or IgG4; hybrid IgG2/4Fc domains can also be used, e.g., an Fc with residues 117-260 from IgG2 and residues 261-447 from IgG4.

In some embodiments, in the protein or polypeptide (e.g., an antibody), the N-terminus of the immunoglobulin variable domain of the present disclosure is linked to the C-terminus of the Fc region (e.g., the CH3 thereof) directly or by a linker. In some embodiments, in the antibody, the N-terminus of the immunoglobulin variable domain of the present disclosure is linked to the C-terminus of the light chain of the antibody (e.g., the CL thereof) directly or by a linker. In the present disclosure, it is not excluded that another immunoglobulin variable domain, Fab, Fab', F(ab')2, Fv, Fab-Fv, Fab-dsFv, single-domain antibody (e.g., VH sdAb or VL sdAb or VHH), scFv, or polypeptide is linked to the N-terminus of the heavy chain and/or the light chain. In some embodiments, the linker is conventional, including, but not limited to, e.g., (GₘSₙ)ₕ or (GGNGT)ₕ or (YGNGT)ₕ or (EPKSS) ₕ, wherein m and n are each independently selected from the group consisting of an integer of 1-8 (e.g., 1, 2, 3, 4, 5, 6, 7, or 8), and h is independently selected from the group consisting of an integer of 1-20 (e.g., 1, 2, 3, 4, 5, 6, 7, 8, 9, 10, 11, 12, 13, 14, 15, 16, 17, 18, 19, or 20). For example, the linker is G₄S, GS, GAP, (G₄S)₂, (G₄S)₃, (G₄S)₄, (G₄S)₅, or ASGS.

In some embodiments, the protein or polypeptide (e.g., an antibody) comprises at least two (e.g., 2, 3, 4, 5, 6, 7, or 8) antigen-binding domains; in some specific embodiments, the at least two (e.g., 2, 3, 4, 5, 6, 7, or 8) antigen-binding domains target two different antigens, or multiple (e.g., 3 or 4) different antigens, or two or more (e.g., 3, 4, 5, or 6) different domains or epitopes of the same antigen.

In some embodiments, the antigens include, but are not limited to, PD-1, PD-L1, CTLA4, TIGIT, CD40, TNFα, TNFR (including TNFR1), VEGF, IL-1R, IL-6R, IL-4, IL-5, IL-13, DC-SIGN, ASGPR, HSA, TGFβR2, and 4-1BB.

In some embodiments, the protein or polypeptide (e.g., an antibody) is therapeutic or prophylactic and is used for treating or preventing diseases/disorders, including, but not limited to, cell proliferative diseases (e.g., tumors), metabolic diseases, autoimmune diseases, or infectious diseases.

In some embodiments, the protein or polypeptide (e.g., an antibody) further comprises a half-life extending domain, e.g., albumin or a polypeptide (HSA), an HSA-binding domain (e.g., an anti-HSA antibody or anti-HSA single-domain antibody), a PEG molecule, or an immunoglobulin Fc region.

The present disclosure also provides a conjugate or derivative comprising the aforementioned sdAb of the present disclosure; or comprising the aforementioned protein or polypeptide of the present disclosure. The conjugate may comprise, e.g., any detectable label.

### Exemplary Immunoglobulin Single Variable Domains

As an example of the aforementioned immunoglobulin single variable domain of the present disclosure, the present disclosure also provides a 4-1BB protein or polypeptide comprising an immunoglobulin single variable domain that specifically binds to 4-1BB. In some embodiments, the immunoglobulin single variable domain that specifically binds to 4-1BB comprises a CDR1, a CDR2, and a CDR3; the CDR1, CDR2, and CDR3 are the CDR1, CDR2, and CDR3 in any one of the amino acid sequences set forth in SEQ ID NOs: 6 and 15-18, and the CDR1, CDR2, and CDR3 are defined according to the Kabat, IMGT, Chothia, AbM, or Contact numbering scheme.

In some embodiments, the immunoglobulin single variable domain that specifically binds to 4-1BB comprises any one of the above CDR1, CDR2, and CDR3 or any combination thereof.

In some embodiments, the amino acid sequences of the CDR1, CDR2, and CDR3 of the immunoglobulin single variable domain that specifically binds to 4-1BB are set forth in SEQ ID NOs: 7, 8, and 9, or SEQ ID NOs: 7, 8, and 18, respectively. The CDRs are defined according to the Kabat numbering scheme.

In some embodiments, the immunoglobulin single variable domain that specifically binds to 4-1BB has undergone humanization, affinity maturation, removal/reduction of T cell epitopes (TCEs), reduction of antibody deamidation, and/or engineering for reducing antibody isomerization. In some specific embodiments, the heavy chain framework regions (FRs) of the human germline template used in the humanization are derived from IGHV3-64*04, IGHV3-23*03, and/or IGHV3-74*01. In some embodiments, FR1 is derived from IGHV3-64*04, FR2 is derived from IGHV3-23*03, and FR3 is derived from IGHV3-74*01.

In some embodiments, the amino acid sequence of the immunoglobulin single variable domain that specifically binds to 4-1BB is set forth in any one of SEQ ID NOs: 6, 15-18, and 20-35 or has at least 90% or at least 95% identity thereto.

In the present disclosure, "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity; "at least 90% (sequence) identity" encompasses at least 90%, at least 91%, at least 92%, at least 93%, at least 94%, at least 95%, at least 96%, at least 97%, at least 98%, at least 99%, or 100% (sequence) identity; "at least 95% (sequence) identity" encompasses at least 95%, at least 96%, at least 97%, at least 98%, at least 99%, or 100% (sequence) identity.

In some embodiments, provided is an immunoglobulin single variable domain that specifically binds to 4-1BB, the amino acid sequence of which is set forth in any one of SEQ ID NOs: 21 and 30-35 and which has reduced binding to pre-ADAs (or ADAs), as compared to a sdAb that specifically binds to 4-1BB, which is set forth in any one of SEQ ID NOs: 20 and 22-29.

In some embodiments, provided is a protein or polypeptide (including an antibody) comprising the aforementioned immunoglobulin single variable domain that specifically binds to 4-1BB. One or more (e.g., 1, 2, 3, 4, 5, or 6) said immunoglobulin single variable domains that specifically bind to 4-1BB may be present, and they may be identical or different. The antibody is, e.g., a mono-, bi-, or multi-specific antibody.

In some embodiments, the aforementioned protein or polypeptide (including an antibody) also comprises a human immunoglobulin Fc region; for example, the Fc region is the Fc region of human IgG1, IgG2, IgG3, or IgG4. In some embodiments, the Fc region is an Fc region with reduced effector function, e.g., reduced ADCC, ADCP, or CDC. In some embodiments, the Fc region is set forth in any one of SEQ ID NOs: 10-12 or has at least 80% or at least 90% sequence identity thereto.

In some embodiments, the aforementioned immunoglobulin single variable domain that specifically binds to 4-1BB has activity selected from the group consisting of at least one of the following:
(a) binding to human 4-1BB or an epitope thereof with a K_{D} value of ≤10⁻⁷;
(b) when not cross-linked by FcyRIIb (i.e., CD32b), having weak or no activation of the 4-1BB signaling pathway; for example, when at a 100 n antibody concentration and not cross-linked by FcyRIIb, having a degree of activation that is no more than 10% of the activity when cross-linked by FcyRIIb and at a saturated antibody concentration;
(c) when cross-linked by FcyRIIb, having relatively strong or strong activation of the 4-1BB signaling pathway with, e.g., an EC₅₀ of less than 1 nM;
(d) activating T cells and/or promoting T cell proliferation; and
(e) inhibiting tumor growth;
wherein the measurement of the activation of the 4-1BB signaling pathway in (b) and (c) is performed, e.g., using the 4-1BB/NF-xB luciferase reporter gene assay method of Example 5.

In some embodiments, the immunoglobulin single variable domain that specifically binds to 4-1BB may bind to 4-1BB with a K_{D} value of ≤1 × 10⁻⁷ M, e.g., ≤1 × 10⁻⁸ M, or ≤1 × 10⁻⁹ M, or ≤1 × 10⁻¹⁰ M.

In some embodiments, the immunoglobulin single variable domain that specifically binds to 4-1BB is capable of inhibiting tumor growth by at least about 10%, e.g., at least about 20%, about 30%, about 40%, about 50%, about 60%, about 70%, or about 80%.

In some embodiments, provided is a protein or polypeptide (e.g., an antibody) that binds to, or competes for binding to the same epitope with, the aforementioned sdAb of the present disclosure that specifically binds to 4-1BB; or that blocks the binding of the aforementioned sdAb of the present disclosure that specifically binds to 4-1BB to 4-1BB; or whose binding to 4-1BB is blocked by the aforementioned sdAb of the present disclosure that specifically binds to 4-1BB.

### Polynucleotide and Vector

Provided is a nucleic acid molecule encoding the aforementioned immunoglobulin single variable domain of the present disclosure or the protein or polypeptide (including an antibody) comprising the immunoglobulin single variable domain. The nucleic acid may be an RNA, DNA, or cDNA. According to some embodiments of the present disclosure, the nucleic acid is a substantially isolated nucleic acid.

The nucleic acid of the present disclosure may also be in the form of a vector, and it may be present in the vector and/or may be part of the vector. The vector is, e.g., a plasmid, cosmid, YAC, or viral vector. The vector may especially be an expression vector, i.e., a vector that provides for the *in vitro* and/or *in vivo* (i.e., in suitable host cells, host organisms, and/or expression systems) expression of a PD-1-binding protein or polypeptide. The expression vector generally comprises at least one of the nucleic acids of the present disclosure, which is operably linked to one or more suitable expression regulatory elements (e.g., promoters, enhancers, and terminators). The selection of the elements and their sequences for expression in a particular host is within the knowledge of those skilled in the art. Regulatory elements and other elements useful or necessary for expression of the sdAb of the present disclosure or the protein or polypeptide (including an antibody) comprising the sdAb include, e.g., promoters, enhancers, terminators, integration factors, selection labels, leader sequences, and reporter genes.

The nucleic acid of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

### Host Cell

Provided is a recombinant host cell that expresses or is capable of expressing the aforementioned immunoglobulin single variable domain of the present disclosure or the protein or polypeptide (including an antibody) comprising the immunoglobulin single variable domain and/or comprises the nucleic acid or vector of the present disclosure. In some embodiments, the host cell is a bacterial cell, a fungal cell or a mammalian cell. Bacterial cells include, e.g., cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, e.g., cells of the species *Trichoderma*, *Neurospora*, and *Aspergillus*; or cells of the species *Saccharomyces* (e.g., *Saccharomyces cerevisiae*), *Schizosaccharomyces* (e.g., *Schizosaccharomyces pombe*), *Pichia* (*Pichia pastoris* and *Pichia methanolica*), and *Hansenula.*

Mammalian cells include, e.g., HEK293 cells, CHO cells, BHK cells, HeLa cells, COS cells, and the like.

However, amphibian cells, insect cells, plant cells, and any other cells used in the art for expressing heterologous proteins or polypeptides may also be used in the present disclosure.

### Preparation Method

Provided is a method for preparing the aforementioned immunoglobulin single variable domain of the present disclosure or the protein or polypeptide (including an antibody) comprising the immunoglobulin single variable domain, comprising: expressing the protein or polypeptide of interest in a host cell as described above, and isolating the protein or polypeptide of interest from the host cell. Optionally, a purification step can also be included. For example, purification is performed using an A or G Sepharose FF column containing an adjusted buffer to wash off non-specifically bound components, and then bound antibodies are eluted by a pH gradient method, detected by SDS-PAGE, and collected. Optionally, the antibody solution can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

The engineered antibody or antigen-binding fragment of the present disclosure can be prepared and purified by conventional methods. For example, cDNA sequences encoding the heavy and light chains can be cloned and recombined into an expression vector. Recombinant immunoglobulin expression vectors can be stably transfected into CHO cells. Mammalian expression systems may result in glycosylation of antibodies, particularly at the highly conserved N-terminus of the Fc region. Stable clones are obtained by expression of antibodies specifically binding to the human-derived antigen. Positive clones are expanded in a serum-free medium of a bioreactor to produce antibodies. The culture solution with the secreted antibody can be purified and collected by conventional techniques. The antibody can be filtered and concentrated by conventional methods. Soluble mixtures and multimers can also be removed by conventional methods, such as molecular sieves and ion exchange.

### Composition

Provided is a composition comprising the aforementioned immunoglobulin single variable domain of the present disclosure or the protein or polypeptide (including an antibody) comprising the immunoglobulin single variable domain. For example, provided is a pharmaceutical composition comprising the immunoglobulin single variable domain or the protein or polypeptide (including an antibody) comprising the immunoglobulin single variable domain of the present disclosure in an amount that is effective in treating, alleviating, or preventing a disease (e.g., a cancer), and at least one pharmaceutically acceptable excipient, diluent, or carrier.

In some specific embodiments, a unit dose of the pharmaceutical composition may comprise 0.01 to 99 weight% of the immunoglobulin single variable domain or the protein or polypeptide (including an antibody) comprising the immunoglobulin single variable domain of the present disclosure, or a unit dose of the pharmaceutical composition comprises the immunoglobulin single variable domain or the protein or polypeptide (including an antibody) comprising the immunoglobulin single variable domain of the present disclosure in an amount of 0.1-2000 mg, and in some embodiments, 1-1000 mg. In some embodiments, provided is a product comprising the aforementioned immunoglobulin single variable domain of the present disclosure or the protein or polypeptide (including an antibody) comprising the immunoglobulin single variable domain. Optionally, the product comprises a container and a label. Examples of containers are bottles, syringes, and test tubes. The container has a composition effective in treating a disorder (e.g., a cancer, an autoimmune disease, or a metabolic disease) contained therein. The label on or associated with the container indicates that the composition is used for treating the disorder of choice (e.g., a cancer, an autoimmune disease, or a metabolic disease).

In some embodiments, provided is a pharmaceutical composition comprising the aforementioned immunoglobulin single variable domain of the present disclosure or the protein or polypeptide (including an antibody) comprising the immunoglobulin single variable domain. The immunoglobulin single variable domain or the protein or polypeptide (including an antibody) comprising the immunoglobulin single variable domain may be in an amount that is effective in treating or alleviating a disease (e.g., a cancer, an autoimmune disease, or a metabolic disease), and the pharmaceutical composition may also comprise at least one pharmaceutically acceptable excipient, diluent, or carrier.

When the immunoglobulin single variable domain is an immunoglobulin single variable domain that specifically binds to 4-1BB, the disorder is, e.g., a cancer, e.g., selected from the group consisting of lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell cancer, hematological cancer, or any combination thereof.

### Treatment Method and Pharmaceutical Use

Provided is a method for treating, alleviating, preventing, or diagnosing a disease or disorder using the aforementioned immunoglobulin single variable domain of the present disclosure, the protein or polypeptide (including an antibody) comprising the immunoglobulin single variable domain, and the pharmaceutical composition thereof. In some embodiments, provided is a method for ameliorating, alleviating, treating, or preventing a disease, comprising administering to a subject the immunoglobulin single variable domain of the present disclosure, the protein or polypeptide (including an antibody) comprising the immunoglobulin single variable domain, and the pharmaceutical composition thereof in an amount that is effective in ameliorating, alleviating, treating, or preventing the disease.

In some embodiments, provided is use of the immunoglobulin single variable domain of the present disclosure, the protein or polypeptide (including an antibody) comprising the immunoglobulin single variable domain, and the pharmaceutical composition thereof for the preparation of a medicament for ameliorating, alleviating, treating, or preventing a disease.

In some embodiments, the aforementioned disease is a proliferative disorder or any other disease or disorder characterized by uncontrolled cell growth (e.g., a cancer; in the present disclosure, cancer and tumor are used interchangeably). In some embodiments, the cancer is a solid tumor or hematological tumor. In some embodiments, the cancer is advanced or metastatic. In some embodiments, the cancer is selected from the group consisting of lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell cancer, hematological cancer, or any combination thereof.

In some embodiments, provided is a method for treating or preventing an infection or autoimmune disease in a subject, comprising administering to the subject a therapeutically or prophylactically effective amount of the immunoglobulin single variable domain of the present disclosure, the protein or polypeptide (including an antibody) comprising the immunoglobulin single variable domain, and the pharmaceutical composition thereof. In some embodiments, the infection is a pathogen infection characterized by different degrees of dysfunction of a virus-specific T cell response, such as HIV, HCV, or HBV. In some embodiments, the autoimmune disease is selected from the group consisting of systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis.

### Detection

Provided is use of the immunoglobulin single variable domain of the present disclosure or the protein or polypeptide (including an antibody) comprising the immunoglobulin single variable domain for *in vivo* or *in vitro* detection.

Also provided is a method, system, or device for detecting side effects (including immunogenicity and binding to ADAs or pre-ADAs) caused by the immunoglobulin single variable domain or the protein or polypeptide (including an antibody) comprising the immunoglobulin single variable domain, comprising treating a sample, e.g., a serum sample taken from a subject, with the immunoglobulin single variable domain of the present disclosure or the protein or polypeptide (including an antibody) comprising the immunoglobulin single variable domain; the subject may be one in whom the immunoglobulin single variable domain of the present disclosure or the protein or polypeptide (including an antibody) comprising the immunoglobulin single variable domain is to be administered to treat or prevent a disease.

In some embodiments, the immunoglobulin single variable domain of the present disclosure or the protein or polypeptide (including an antibody) comprising the immunoglobulin single variable domain is used in a method for predicting whether it gives rise to protein or polypeptide interference, e.g., for determining whether the immunoglobulin single variable domain or the protein or polypeptide (including an antibody) comprising the immunoglobulin single variable domain has a relatively high or increased risk of giving rise to protein or polypeptide interference, or binding to an interfering factor (e.g., pre-ADAs) present in human blood or serum.

In some embodiments, provided is a method for predicting whether an immunoglobulin single variable domain or a protein or polypeptide (including an antibody) comprising the immunoglobulin single variable domain will give rise to protein or polypeptide interference in an immunoassay (such as an ADA or pre-ADA assay), the method comprising performing an immunoassay comprising at least the following steps:
(i) bringing said any immunoglobulin single variable domain or protein or polypeptide comprising the immunoglobulin single variable domain (including an antibody) into contact with an antibody that has been obtained from a human subject and that has been selected/isolated based on its capacity to recognize and/or bind to the C-terminus of an immunoglobulin single variable domain that ends with the amino acid sequence VTVSS at the C-terminus (the "unmodified immunoglobulin single variable domain" in the present disclosure); and
(ii) determining whether said any immunoglobulin single variable domain or protein or polypeptide (including an antibody) comprising the immunoglobulin single variable domain binds to the antibody in the immunoassay. Binding to the antibody in step (ii) means that said any immunoglobulin single variable domain or protein or polypeptide (including antibody) comprising the immunoglobulin single variable domain is capable of giving rise to (or has a relatively high or increased risk of giving rise to) such protein or polypeptide interference.

In some embodiments, provided is a method for predicting the extent to which an immunoglobulin single variable domain or a protein or polypeptide or antibody comprising the immunoglobulin single variable domain gives rise to protein or polypeptide interference in an immunoassay, the method comprising performing an immunoassay comprising at least the following steps:
(i) bringing any immunoglobulin single variable domain or protein or polypeptide comprising the immunoglobulin single variable domain (including an antibody) of the present disclosure into contact with an antibody that has been obtained from a human subject and that has been selected/isolated based on its capacity to recognize and/or bind to the C-terminus of an immunoglobulin single variable domain that ends with the amino acid sequence VTVSS at its C-terminus; and
(ii) determining whether the immunoglobulin single variable domain or the protein or polypeptide (including an antibody) comprising the immunoglobulin single variable domain binds to the antibody in the immunoassay, and the extent of binding (in some embodiments, the extent of binding is compared to the extent to which an immunoglobulin single variable domain that ends with the amino acid sequence VTVSS at the C-terminus binds to the antibody under the same experimental or equivalent detection conditions);
wherein the presence of binding or the extent of binding being relatively high in step (ii) means that the immunoglobulin single variable domain or the protein or polypeptide (including an antibody) comprising the immunoglobulin single variable domain is capable of giving rise to protein or polypeptide interference, or has a relatively high or increased risk of giving rise to protein or polypeptide interference; the absence of binding or the extent of binding being relatively low in step (ii) means that the immunoglobulin single variable domain or the protein or polypeptide (including an antibody) comprising the immunoglobulin single variable domain does not give rise to protein or polypeptide interference, or has a relatively low risk of giving rise to protein or polypeptide interference; for example, the immunoassay is an ELISA assay.

In some specific embodiments, the antibody is a polyclonal antibody. In some embodiments, the antibody is a polyclonal antibody that can be obtained from a biological sample that has been obtained from a subject and is suitable for use as a starting material for obtaining a polyclonal antibody by using a method comprising at least one immunoaffinity chromatography step and optionally one or more additional steps for separating and/or purifying a polyclonal antibody from the sample; in the immunoaffinity chromatography, an immunoglobulin single variable domain or a protein or polypeptide (including an antibody) comprising the immunoglobulin single variable domain is used as an affinity moiety or antigen, wherein the immunoglobulin single variable domain used as the affinity moiety or antigen ends with the amino acid sequence VTVSS at its C-terminus.

In some embodiments, provided is a method for reducing the tendency of an immunoglobulin single variable domain that ends with the sequence VTVSS at its C-terminus (i.e., the "unmodified immunoglobulin single variable domain" in the present disclosure) or a protein or polypeptide (including an antibody) comprising the sdAb to give rise to protein or polypeptide interference in an anti-drug antibody assay; or provided is use of a C-terminal modification for reducing the tendency of an immunoglobulin single variable domain or a protein or polypeptide (including an antibody) comprising the sdAb to give rise to protein or polypeptide interference, comprising using the aforementioned method provided by the present disclosure to modify the C-terminus of the immunoglobulin single variable domain so that its tendency to give rise to protein or polypeptide interference is reduced or substantially reduced, as compared to an immunoglobulin single variable domain that has the sequence VTVSS at its terminus (i.e., the "unmodified immunoglobulin single variable domain" in the present disclosure).

In some embodiments, the method, system, or device for *in vitro* detection may comprise, e.g.,
(i) bringing a sample into contact with the immunoglobulin single variable domain of the present disclosure or the protein or polypeptide (including an antibody) comprising the immunoglobulin single variable domain;
(ii) detecting a complex formed between any immunoglobulin single variable domain or protein or polypeptide (including an antibody) comprising the immunoglobulin single variable domain and the sample; and/or
(iii) bringing a reference sample (e.g., a control sample) into contact with any immunoglobulin single variable domain or protein or polypeptide (including an antibody) comprising the immunoglobulin single variable domain; and
(iv) determining the extent of complex formation by comparison with the reference sample. A change (e.g., a statistically significant change) in complex formation in the sample or subject as compared to that in the control sample or subject indicates the extent to which said any immunoglobulin single variable domain or protein or polypeptide (including an antibody) comprising the immunoglobulin single variable domain binds to pre-ADAs (or ADAs) in the subject from whom the sample is taken.

The detection may include determining the location or time at which the complex is formed. The immunoglobulin single variable domain or the protein or polypeptide (including an antibody) comprising the immunoglobulin single variable domain is also labeled with a detectable substance, and detection is achieved through the label. Suitable detectable substances include various enzymes, prosthetic groups, fluorescent substances, luminescent substances, and radioactive substances. Conventional detection assays may be used, such as enzyme-linked immunosorbent assay (ELISA), radioimmunoassay (RIA), or tissue immunohistochemistry. For detection purposes, the sdAb of the present disclosure and the protein or polypeptide (including an antibody) comprising the immunoglobulin single variable domain may be labeled with a fluorophore chromophore. In some embodiments, also provided is a kit, comprising the aforementioned binding protein or polypeptide or polynucleotide; the kit may also comprise instructions for diagnostic use. The kit may also comprise at least one additional reagent, such as a label or an additional diagnostic agent. For *in vivo* use, the antibody may be formulated into a pharmaceutical composition.

The assay method of WO2012175741A is incorporated into the present disclosure by reference in its entirety.

### Definitions of Terms

In order to facilitate the understanding of the present disclosure, some technical and scientific terms are specifically defined below. Unless otherwise specifically defined in the present disclosure, all other technical and scientific terms used in the present disclosure have the meanings generally understood by those of ordinary skill in the art to which the present disclosure pertains.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to".

The three-letter and single-letter codes for amino acids used herein are as described in J. biol. chem, 243, p3558 (1968).

"4-1BB protein or polypeptide" or "4-1BB", also known as CD137 or tumor necrosis factor receptor superfamily 9, is a member of the TNF receptor superfamily (TNFRSF) and a costimulatory molecule expressed on the cell surface of CD8⁺ and CD4⁺ T cells, regulatory T cells (Tregs), NK cells, and NKT cells, B cells, and neutrophils and the like. 4-1BB is a costimulatory molecule expressed upon activation of immune cells. Human 4-1BB protein or polypeptide is under the NCBI accession number NP_001552.2. In the present disclosure, "4-1BB" may optionally include any protein or polypeptide of this kind, or fragments or variants thereof, including (but not limited to) the known or wild-type 4-1BB described in the present disclosure, as well as any naturally occurring splice variants, amino acid variants, or isoforms.

"Antibody" or "immunoglobulin" is used in the broadest sense and encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. "Antigen-binding fragment" includes, but is not limited to: a single-chain antibody (i.e., full-length heavy and light chains); a Fab, a modified Fab, a Fab', a modified Fab', an F(ab')2, an Fv, a Fab-Fv, a Fab-dsFv, a single-domain antibody (e.g., VH/VL or VHH), an scFv, a bivalent or trivalent or tetravalent antibody, a Bis-scFv, a diabody, a tribody, a triabody, a tetrabody and an epitope-binding fragment of any one of the above (see, e.g., Holliger and Hudson, 2005, Nature Biotech. 23(9): 1126-1136; Adair and Lawson, 2005, Drug Design Reviews-Online 2(3), 209-217).

"Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including a VHH, humanized VHH, camelized VH (e.g., a camelized human VH)), IgNAR, domains, (single-domain) antibodies as VH domains or derived from VH domains (such as dAbs^{™}) and (single-domain) antibodies as VL domains or derived from VL domains (such as dAbs^{™}). Immunoglobulin single variable domains based on and/or derived from heavy chain variable domains (such as VH or VHH domains) are generally preferred. A specific example of an immunoglobulin single variable domain is a "VHH domain" (or simply "VHH") as defined below. In the present disclosure, domain antibody (dAb) and single-domain antibody (sdAb) are used interchangeably.

"VHH", also known as nanobody, VHH antibody, VHH antibody fragment, VHH domain, or heavy-chain single-domain antibody (sometimes also referred to as single-domain antibody), refers to the variable region of a heavy-chain antibody (Hamers-Casterman C, Atarhouch T, Muyldermans S, Robinson G, Hamers C, Songa EB, Bendahman N, Hamers R.: "Naturally occurring antibodies devoid of light chains"; Nature363, 446-448(1993)). The use of "VHH" aims to distinguish it from the heavy chain variable regions (VHs) in conventional four-peptide-chain antibodies. The domain specificity of VHHs enables them to bind alone to epitopes without the need for additional antigen-binding domains. For conventional four-peptide-chain antibodies, recognition of an epitope involves both the VL domain and the VH domain. A VHH domain is a small, stable, and efficient antigen recognition unit formed by a single immunoglobulin domain. In some embodiments, taking 4-1BB as an example, "anti-4-1BB single-domain antibody", "4-1BB VHH", "4-1BB VHH antibody", and "4-1BB nanobody" are used interchangeably.

A VHH generally consists of 110-120, e.g.,112-115, amino acids. It should be noted, however, that longer and shorter sequences may also be suitable for the purposes described in the present disclosure. Other structural and functional properties of VHHs and VHH-containing polypeptides can be summarized as follows: in the absence of a VL and without interaction with a VL, a VHH functionally binds to an antigen. The VH and VL domains of conventional four-peptide-chain antibodies themselves are not suitable for practical use as individual antigen-binding molecules or immunoglobulin single variable domains, and they need to be in some form or combined to provide a functional antigen-binding unit (e.g., in the form of a Fab or scFv). A VHH can be used as an individual and relatively small functional antigen-binding unit, domain, or polypeptide. Due to these distinctive properties, the use of VHH domains (alone or as part of a larger polypeptide) has many significant advantages over the use of conventional VH and VL domains, scFv or conventional antibody fragments (such as Fab or F(ab')2 fragments):
- only a single domain is required to bind to an antigen with high affinity and high selectivity, so that there is no need to have two separate domains present, nor to assure that these two domains are present in the right spatial conformation and configuration (e.g., the use of specially designed linker is generally required for an scFv);
- VHH domains can be expressed from a single gene and do not require post-translational folding or modification;
- VHH domains can be easily engineered into multivalent and multi-specific formats;
- VHH domains are highly soluble and do not have a tendency to aggregate;
- VHH domains are highly stable to heat, pH, protein or polypeptide enzymes, and other denaturing agents or conditions and, thus, may be prepared, stored, or transported without the use of refrigeration equipment, so that the cost, time, and environment can be saved;
- VHH domains are easy to prepare and relatively inexpensive, even on a manufacturing scale;
- VHH domains are relatively smaller than conventional four-peptide-chain antibodies and antigen-binding fragments thereof (about 15 kDa or 1/10 of the size of a conventional IgG), and therefore show relatively high tissue permeability and can be administered at relatively high doses;
- VHH domains can show so-called cavity-binding properties (compared to conventional VH domains, VHHs have extended CDR3 loops, so that they can access target epitopes not accessible to conventional four-peptide-chain antibodies and antigen-binding fragments thereof).

Methods for obtaining VHHs that bind to target antigens or epitopes have previously been disclosed in the following documents: R. van der Linden et al., Journal of Immunological Methods, 240(2000)185-195; Li et al., J Biol Chem., 287(2012)13713-13721; Deffar et al., African Journal of Biotechnology Vol.8(12), pp.2645-2652, 17 June, 2009, and WO94/04678.

A VHH domain can be "humanized" (also referred to as "sequence optimization" in the present disclosure; in addition to humanization, "sequence optimization" may also encompass other modifications that provide improved properties for the VHH, such as the removal of potential post-translational modification sites) by substituting one or more amino acid residues in the amino acid sequence of the original VHH (for example, a VHH derived from the family Camelidae) with one or more amino acid residues at corresponding positions in the VH domain of a conventional human four-peptide chain antibody. The humanized VHH domain may contain one or more fully human framework region sequences, and in one specific embodiment, may contain the human framework region sequence of IGHV3. Humanization methods include, e.g., resurfacing, CDR grafting to a universal framework, Framework shuffling, and the like. Human FR germline sequences are available, e.g., from the ImMunoGeneTics (IMGT) website.

"Domain" (of a protein or polypeptide) refers to a folded protein or polypeptide structure. In general, a domain is responsible for a single function of a protein or polypeptide. In many cases, a domain can be added, deleted, or transferred to another protein or polypeptide without losing the functions of the other parts and/or domains of the protein or polypeptide. In some embodiments of the present disclosure, "domain" may also be used as a site where an antibody binds to an antigen.

"Variable domain" refers to a domain essentially consisting of four "framework regions" referred to in the art and the context as "framework region 1" (FR1), "framework region 2" (FR2), "framework region 3" (FR3), and "framework region 4" (FR4), wherein the framework regions are interrupted by three "complementarity-determining regions" referred to in the art and the context as "complementarity-determining region 1" (CDR1), "complementarity-determining region 2" (CDR2), and "complementarity-determining region 3" (CDR3). Thus, the general structure (or sequence) of a variable domain can be expressed as follows: FR1-CDR1-FR2-CDR2-FR3-CDR3-FR4. A variable domain possesses specificity for an antigen by virtue of having an antigen-binding site.

"Framework region (FR)" or "framework" refers to a portion of a variable domain that serves as a framework for the CDRs.

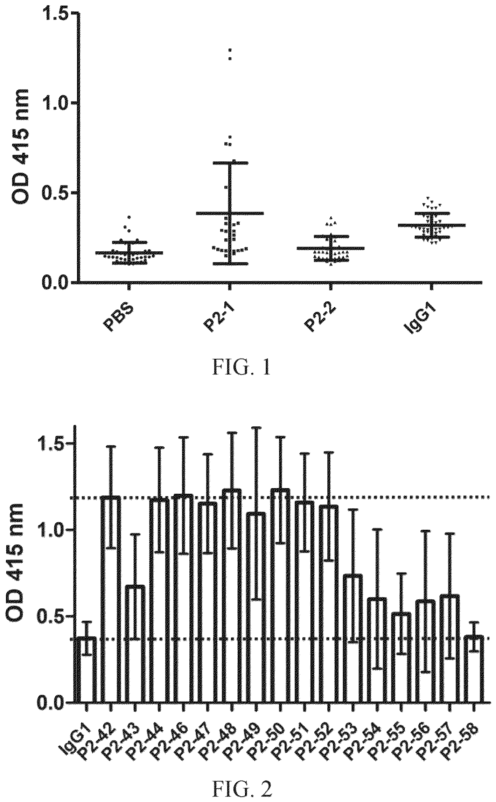

For example, as shown in FIG. 2 of Riechmann and Muyldermans, J. Immunol. Methods 231, 25-38(1999), amino acid residues employed for VHH domains are numbered according to the general numbering scheme for VH domains proposed by Kabat et al ("Sequence of proteins of immunological interest", US Public Health Services, NIH Bethesda, MD, Publication No. 91). It should be noted, however, that as is well known in the art for VH domains and VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence. One skilled in the art can determine the amino acid sequence boundaries of antibody CDRs using any one of many known numbering schemes, including the numbering schemes described in the following documents: Kabat et al., supra (the "Kabat" numbering scheme); Al-Lazikani et al., 1997, J. Mol. Biol., 273:927-948 (the "Chothia" numbering scheme); MacCallum et al., 1996, J. Mol. Biol., 262:732-745 (the "Contact" numbering scheme); Lefran et al., Dev. Comp. Immunol., 2003,27:55-77 ("IMGT" numbering scheme), and Honegge and Plückthun, J. Mol. Biol., 2001,309:657-70 (the "AHo" numbering scheme), each of which is incorporated herein by reference in its entirety. Alternative methods for numbering the amino acid residues of VH domains are known in the art, and the alternative methods may also be applied similarly to VHH domains. However, in the specification, claims, and drawings of the present disclosure, the numbering according to Kabat and appropriate for VHH domains described above will be followed, unless otherwise stated.

"Humanized" antibody refers to an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portions of the variable region).

An "affinity-matured" antibody, in particular a VHH or domain antibody, has one or more changes in one or more CDRs that result in increased affinity for a target antigen as compared to its parent antibody. Affinity-matured antibodies may be prepared by methods known in the art.

"Fc region" or "fragment crystallizable region" is used to define the C-terminal region of the heavy chain of an antibody, including native Fc regions and modified Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

"Unmodified immunoglobulin single variable domain", e.g., an immunoglobulin single variable domain that binds to a target antigen, comprises three complementarity-determining regions (CDRs) within a framework structure. Whereas in the genetics of naturally occurring immunoglobulin chains, the V region ends at the beginning of CDR3, and the remainder of CDR3 is provided by the D and J regions (resulting in a V-D-J fusion), the immunoglobulin single variable domain of the present disclosure comprises all of CDR3 and ends with FR4 residues at its C-terminus. A VH sdAb ends with the residues LVTVSS at its C-terminus. A VHH sdAb ends with the residues VTVSS at its C-terminus. A VL sdAb ends with VEIKR at its C terminus. "Modified immunoglobulin single variable domain" refers to an immunoglobulin single variable domain as described in the present disclosure that additionally has a modification that alters the three-dimensional conformation of the C-terminus of the immunoglobulin single variable domain. Modified immunoglobulin single variable domains include immunoglobulin single variable domains that comprise C-terminal additions, extensions or tags and/or certain amino acid substitutions as disclosed herein.

"Pre-anti-drug antibody" or "pre-ADA" is an ADA already present in the subject or individual who is to be administered or given a drug (e.g., a protein or polypeptide drug, more e.g., an antibody drug). A pre-anti-drug antibody may be present in an experiment-naive subject or individual (i.e., a subject or individual to whom the drug has never been administered before).

"Antigen" refers to a molecule used for immunization of an immunocompetent vertebrate to produce an antibody that recognizes the antigen or to screen an expression library (e.g., particularly phage, yeast, or ribosome display library). Herein, the antigen is defined in a broader sense, and includes a target molecule that is specifically recognized by the antibody, and a portion or a mimic of a molecule used in an immunization process for producing the antibody or in library screening for selecting the antibody.

"Epitope" or "antigenic determinant" used interchangeably herein refers to any antigenic determinant on an antigen to which an antibody binds. The antigenic determinant generally comprises chemically active surface groups of molecules such as amino acids or sugar side chains, and usually has specific three-dimensional structural characteristics and/or specific charge characteristics. An epitope may be a "linear" epitope or a "conformational" epitope. In a linear epitope, all points of interaction between an antigen and an interacting molecule (e.g., an antibody) exist linearly along the primary amino acid sequence of the antigen. In a conformational epitope, the points of interaction exist across amino acid residues that are separated from one another.

"Specificity" refers to the number of different types of antigens or epitopes to which a target antigen-binding molecule or antigen-binding molecule can bind. The specificity of an antigen-binding molecule can be determined based on its affinity and/or avidity. Affinity, expressed as the dissociation equilibrium constant (K_{D}) between an antigen and an antigen-binding molecule, is a measure of the strength of binding between an epitope and an antigen-binding site on the antigen-binding molecule: the smaller the K_{D} value, the stronger the binding strength between the epitope and the antigen-binding molecule (or affinity can also be expressed as the binding constant (KA), which is 1/K_{D}). As will be appreciated by those skilled in the art, affinity can be determined in a known manner depending on the specific antigen of interest. Avidity is a measure of the strength of binding between an antigen-binding molecule (e.g., an immunoglobulin, an antibody, an immunoglobulin single variable domain, or a polypeptide comprising same) and a related antigen. Avidity is related to both the affinity for its antigen-binding site on the antigen-binding molecule and the number of related binding sites present on the antigen-binding molecule.

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant (K_{D}). K_{D} can be determined by measuring the kinetics of complex formation and dissociation by using, e.g., the surface plasmon resonance (SPR) method (Biacore). The rate constants corresponding to the association and dissociation of a monovalent complex are referred to as the association rate constant kₐ (or kₒₙ) and the dissociation rate constant k_{d} (or k_{off}), respectively. K_{D} is related to kₐ and k_{d} by the equation K_{D} = k_{d}/kₐ. The value of the dissociation constant can be determined directly by well-known methods and can be calculated by methods such as those described by Caceci et al (1984, Byte 9:340-362) even for complex mixtures. For example, K_{D} can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA, and flow cytometry, as well as other assays exemplified elsewhere in the present disclosure. The binding kinetics and binding affinity of the antibody can also be evaluated by standard assays known in the art, such as surface plasmon resonance (SPR), e.g., by using the Biacore^{™} system or KinExA. The binding affinities associated with different molecular interactions, e.g., the binding affinities of different antibodies for a given antigen, can be compared by comparing the K_{D} values of antibody/antigen complexes. Similarly, the specificity of an interaction can be evaluated by determining and comparing the K_{D} value for the interaction of interest (e.g., a specific interaction between an antibody and a target antigen) with the K_{D} value for an interaction not of interest (e.g., a control antibody known not to bind to the target antigen).

"Homology" or "identity" refers to sequence similarity between two polynucleotide sequences or between two polypeptides. When positions in two compared sequences are occupied by identical bases or amino acid monomer subunits, e.g., if the position of each of two DNA molecules is occupied by adenine, the molecules are homologous at that position. The homology percentage between two sequences is a function of the number of matching or homologous positions shared by the two sequences divided by the number of positions compared × 100%. For example, in the optimal alignment of sequences, if 6 out of 10 positions of two sequences are matched or homologous, the two sequences are 60% homologous, and if 95 out of 100 positions of two sequences are matched or homologous, the two sequences are 95% homologous.

"Variant" refers to a nucleotide sequence and amino acid sequence having a different degree of percent sequence identity to the nucleotide sequence and amino acid sequence of the present disclosure, where appropriate mutational modifications such as replacements, insertions, or deletions are made to the nucleotide sequence and amino acid sequence of the present disclosure. The sequence identity may be at least 85%, 90%, or 95%; non-limiting examples include at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100%. Sequence comparison and percent identity determination between two sequences can be performed by the default settings for the BLASTN/BLASTP algorithm available on the website National Center For Biotechnology Institute.

"Conservative substitution" refers to a substitution with another amino acid residue having similar properties to the original amino acid residue. It has little or substantially no effect on the function, activity, or other biological properties of the polypeptide. The conservative amino acid replacements are well known in the art; for example, conservative amino acid substitutions are preferably substitution of one amino acid from the following groups (i)-(v) with another amino acid residue from the same group:
(i) relatively small, aliphatic, nonpolar or weakly polar residues: Ala, Ser, Thr, Pro, and Gly;
(ii) polar, negatively charged residues and (uncharged) amides thereof: Asp, Asn, Glu, and Gln;
(iii) polar, positively charged residues: His, Arg, and Lys;
(iv) relatively large, aliphatic, nonpolar residues: Met, Leu, Ile, Val, and Cys; and
(v) aromatic residues: Phe, Tyr, and Trp.

Particularly preferably, conservative amino acid substitutions are as follows: substitution of Ala with Gly or Ser; substitution of Arg with Lys; substitution of Asn with Gln or His; substitution of Asp with Glu; substitution of Cys with Ser; substitution of Gln with Asn; substitution of Glu with Asp; substitution of Gly with Ala or Pro; substitution of His with Asn or Gin; substitution of Ile with Leu or Val; substitution of Leu with Ile or Val; substitution of Lys with Arg, Gln, or Glu; substitution of Met with Leu, Tyr, or Ile; substitution of Phe with Met, Leu, or Tyr; substitution of Ser with Thr; substitution of Thr with Ser; substitution of Trp with Tyr; substitution of Tyr with Trp or Phe; and substitution of Val with Ile or Leu.

"Back mutation" refers to the mutation of a framework region amino acid residue derived from a human antibody to an amino acid residue at a corresponding position of the original source antibody, which is usually performed to prevent a reduction in activity while the humanization of the antibody causes a reduction in immunogenicity. The humanized antibody variable region may be subjected to minimal reverse mutation to maintain the activity of the antibody. In the present disclosure, "back mutation" also includes mutations in the CDRs.

"Polynucleotide" or "nucleic acid molecule" encompasses DNA and RNA; it may be single-stranded or double-stranded and is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

"Vector" refers to a nucleic acid molecule capable of transporting another nucleic acid to which it has been linked. In one embodiment, the vector is a "plasmid" that refers to a circular double-stranded DNA loop into which additional DNA segments may be ligated.

In another embodiment, the vector is a viral vector, wherein additional DNA segments may be ligated into the viral genome. The vector in the present disclosure is capable of autonomously replicating in a host cell into which they have been introduced (e.g., bacterial vectors having a bacterial origin of replication and episomal mammalian vectors) or being integrated into the genome of a host cell upon introduction into the host cell and thereby replicated along with the host genome (e.g., non-episomal mammalian vectors).

"Cell", "cell line", and "cell culture" are used interchangeably, and all such designations include progeny. Therefore, "transformant" and "transformed cell" include primary test cells and cultures derived therefrom, regardless of the number of transfers. It should also be understood that all progenies may not be precisely identical in DNA content due to intentional or unintentional mutations. Mutant progeny with identical function or biological activity as screened in the original transformed cells is included. When referring to different designations, they will become clear through the context.

"Host cell" refers to a cell into which an expression vector has been introduced. Host cells may include microbial (e.g., bacterial), plant or animal cells. Bacteria susceptible to transformation include members of the *Enterobacteriaceae* family, such as strains of *Escherichia coli* or *Salmonella*; members of the *Bacillaceae* family, such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell line), NS0 cells, and 293 cells. "Pharmaceutical composition" refers to a mixture containing one or more of the compounds or the physiologically/pharmaceutically acceptable salts or prodrugs thereof described in the present disclosure, and other chemical components, for example, physiologically/pharmaceutically acceptable carriers and excipients. The pharmaceutical composition is intended to promote administration to an organism and facilitate the absorption of the active ingredient so that it can exert its biological activity.

"Pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as a phosphate buffered saline solution, water, an emulsion such as an oil/water emulsion, and various types of wetting agents. In some embodiments, the diluent for aerosol or parenteral administration is phosphate buffered saline (PBS) or normal (0.9%) saline. Compositions containing such carriers are formulated by well-known conventional methods (see, e.g., Remington's Pharmaceutical Sciences, 18th edition, A. Gennaro, eds., Mack Publishing Co., Easton, PA, 1990; and R Remington, The Science and Practice of Pharmacy, 20th edition, Mack Publishing, 2000).

"Giving", "administering", and "treating", when applied to animals, humans, subjects, cells, tissues, organs, or biological fluid, refer to contact of an exogenous drug, a therapeutic agent, a diagnostic agent, or a composition with the animals, humans, subjects, cells, tissues, organs, or biological fluid. "Giving", "administering", and "treating" can refer to, e.g., therapeutic, pharmacokinetic, diagnostic, research, and experimental methods. The treatment of cells comprises contacting the reagent with the cells and contacting the reagent with fluid, where the fluid is in contact with the cells. "Giving", "administering", and "treating" also refer to treating a cell, by a reagent, diagnosis, a binding composition, or by another cell *in vitro* and *ex vivo.* "Treating", when applied to humans, veterinary or research subjects, refers to therapeutic treatment, preventive or prophylactic measures, and research and diagnostic applications.

"Effective amount" comprises an amount sufficient to ameliorate or prevent a symptom or sign of a medical disorder. An effective amount also refers to an amount sufficient to allow or facilitate diagnosis. The effective amount for a subject may vary depending on factors such as the disorder to be treated, the general health of the subject, the method and dose of administration, and the severity of side effects. An effective amount may be the maximum dose or administration regimen to avoid significant side effects or toxic effects. The subject of the present disclosure may be an animal or a human subject.

"Subject" or "patient" means a mammal, particularly a primate, and particularly a human. "Treating" or "treatment" means administering internally or externally to a subject a therapeutic agent, e.g., a composition comprising any one of the antibodies or antigen-binding fragments thereof of the present disclosure or a nucleic acid molecule encoding an antibody or an antigen-binding fragment thereof; the patient has one or more disease symptoms on which the therapeutic agent is known to have a therapeutic effect. Generally, the therapeutic agent is administered in an amount effective to alleviate one or more symptoms of the disease in the patient or population being treated to induce regression of such symptoms or to inhibit the development of such symptoms to any clinically measurable degree. The amount of therapeutic agent effective to alleviate any particular symptom of the disease (also known as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example, treatment methods or products) may not be effective in alleviating all the target disease symptoms in every patient, as determined according to any statistical testing methods known in the art, such as Student t-test, chi-square test, Mann and Whitney's U test, Kruskal-Wallis test (H test), Jonckheere-Terpstra test and Wilcoxon test, they should reduce the target disease symptoms in a statistically significant number of patients.

"Sequence" (e.g., in "immunoglobulin sequence", "antibody sequence", "single variable domain sequence", "VHH sequence", or "protein or polypeptide sequence") is generally intended to include both related amino acid sequences and nucleic acid or nucleotide sequences encoding the sequences, unless a further limited interpretation is required in the present disclosure.

"Optional" or "optionally" means that the event or circumstance subsequently described may, but does not necessarily, occur, and this description includes instances where the event or circumstance occurs or does not occur.

### BRIEF DESCRIPTION OF THE DRAWINGS

FIG. 1 shows the frequency of pre-ADAs in serum samples taken from healthy individuals.
FIG. 2 shows the effects of different amino acid modification methods on the binding of the sdAb to pre-ADAs.
FIG. 3 shows the frequency of pre-ADAs for sdAbs with amino acid modifications in serum samples taken from healthy individuals.
FIG. 4 shows the FACS assay results for the binding of the test antibodies to the human 4-1BB antigen.
FIG. 5 shows the 4-1BB/NF-κB luciferase reporter gene assay results for the test antibodies before FcyRIIb cross-linking (A of FIG. 5) and after cross-linking (B of FIG. 5).
FIG. 6 shows the FACS assay results for the binding of the anti-4-1BB single-domain antibody C5 and its humanized antibodies C5_V1, C5_V2, and C5_V3 to human 4-1BB on the surface of HEK293 cells, with urelumab as a control.
FIG. 7 shows the activation of the NF-κB signaling pathway by the anti-4-1BB single-domain antibody C5 and its humanized antibodies C5_V1, C5_V2, and C5_V3, with urelumab and isotype IgG1 antibody as controls.
FIG. 8 shows the FACS assay results for the binding of C5_V2 and C5_V2-YTI to human 4-1BB on the surface of HEK293 cells, with urelumab and isotype IgG1 antibody as controls.
FIG. 9 shows the binding of different amino acid modification variants of C5_V2-YTI to pre-ADAs.

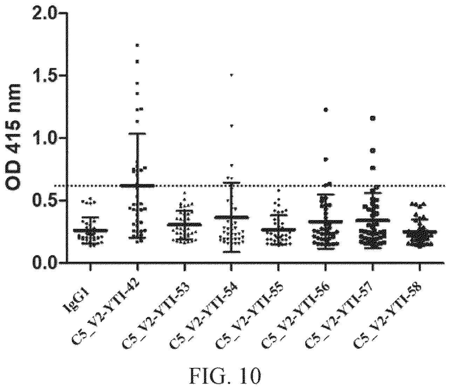

FIG. 10 shows the frequency of pre-ADAs for amino acid modification variants of C5_V2-YTI in serum samples taken from healthy individuals.

### DETAILED DESCRIPTION

The present disclosure is further described below with reference to examples, but these examples are not intended to limit the scope of the present disclosure.

Experimental methods without specific conditions indicated in the examples or test examples are generally conducted according to conventional conditions, or according to conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents.

### Example 1. pre-ADA Assay of Single-Domain Antibodies (sdAbs)

In the present disclosure, an enzyme-linked immunosorbent assay (ELISA) was performed to measure the pre-ADA effects of single-domain antibodies. ELISAs are commonly used to measure the pre-ADA effects of antibodies. The serum samples used in this assay were individual serum samples taken from healthy humans (Shanghai Xinfan Biotechnology Co., Ltd.), and the secondary antibody used was an anti-human antibody λ chain HRP-labeled antibody (Southern Biotech, 2070-05).

According to previous studies, we synthesized the antibody sequences of P2-1 and P2-2, which were derived from the sdAbs (sequence 1 and sequence 16) in the GSK patent WO2013024059A. P2-1 was the sequence 1 in WO2013024059A with a His₆ tag fused to the N-terminus, and P2-2 was the sequence 16 in WO2013024059A with a His₆ tag fused to the N-terminus. The corresponding sequence numbers of P2-1 and P2-2 in the present disclosure are SEQ ID NO: 1 and SEQ ID NO: 2. Moreover, we synthesized an IgG1 molecule the amino acid sequence of which is identical to that of cetuximab. The heavy and light chain amino acid sequences of the IgG1 molecule are set forth in SEQ ID NO: 3 and SEQ ID NO: 4, respectively.

The ELISA method was as follows:
1) Antigen coating: Antibody samples to be tested, including P2-1 and P2-2, were diluted with PBS to an effective final concentration of 2 ng/µL and added to a 96-well plate at 50 µL/well, and the plate was incubated at room temperature for 1 h.
2) Blocking: The supernatant was removed, and the plate was washed with PBST once. A 4% M-PBST blocking solution was added at 150 µL/well, and the plate was incubated at room temperature for 1 h.
3) Serum incubation: The plate was washed with PBST once. The individual serum samples (diluted with PBS in a 1:5 ratio) were added at 30 µL/well, and the plate was incubated at room temperature for 1 h.
4) Washing: The supernatant was removed, and the plate was washed with PBST 8 times.
5) Secondary antibody incubation: A dilution of the secondary antibody (diluted with 4% M-PBST; see corresponding instructions for the dilution ratio) was added at 50 µL/well, and the plate was incubated at room temperature for 1 h.
6) Washing: The supernatant was removed, and the plate was washed with PBST 8 times.
7) Color development: 50 µL of the substrate ABTS was added, and the plate was incubated in the dark for about 20 min. Then OD415 was measured using SpectraMax iD3 (Molecular Devices).

In this assay, the background reactivity of serum samples taken from a total of 40 healthy donors to each antibody sample was tested, and a negative control PBS group, a P2-1 group, a P2-2 group, and an IgG1 group were set up. The results are shown in FIG. 1. The results show that compared to the negative control PBS group, about 57.5% of the serum samples in the P2-1 group had detectable sdAb-reactive pre-ADAs, whereas about 12.5% of the serum samples in the P2-2 group, which involved engineering for reducing immunogenicity, had detectable sdAb-reactive pre-ADAs. The results are substantially consistent with the data presented in the GSK literature. The pre-ADA positives in the present disclosure mean that samples with a difference of +50% or higher between the corresponding single point signals of the test sample group and the IgG1 group are considered positive samples; otherwise, they are considered negative samples. This applies hereinafter.
> P2-1 (SEQ ID NO: 1) 
HHHHHHGGGGSEVQLLESGGGLVQPGGSLRLSCAASGFTFAHETMVWVRQAP
GKGLEWVSHIPPDGQDPFYADSVKGRFTISRDNSKNTLYLQMNSLRAEDTAVYH
CALLPKRGPWFDYWGQGTLVTVSS
> P2-2 (SEQ ID NO: 2) 
HHHHHHGGGGSEVQLLESGGGLVQPGGSLRLSCAASGFTFAHETMVWVRQAP
GKGLEWVSHIPPDGQDPFYADSVKGRFTISRDNSKNTLYLQMNSLRAEDTAVYH
CALLPKRGPWFDYWGQGTLVTVSSA
> The heavy chain of the IgG1 molecule (SEQ ID NO: 3) 
QVQLKQSGPGLVQPSQSLSITCTVSGFSLTNYGVHWVRQSPGKGLEWLGVIWS
GGNTDYNTPFTSRLSINKDNSKSQVFFKMNSLQSNDTAIYYCARALTYYDYEFA
YWGQGTLVTVSAASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK
> The light chain of the IgG1 molecule (SEQ ID NO: 4)

DILLTQSPVILSVSPGERVSFSCRASQSIGTNIHWYQQRTNGSPRLLIKYASESISGI
PSRFSGSGSGTDFTLSINSVESEDIADYYCQQNNNWPTTFGAGTKLELKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC

### Example 2. Amino Acid Engineering of VH Frameworks (FRs) of Single-Domain Antibodies

To investigate whether the binding of pre-ADAs to the frameworks of single-domain antibodies could be reduced, we selected single-domain antibody P2-42 as the parent clone and made amino acid modifications (including additions, substitutions, and deletions) in the framework regions of P2-42 using site-directed mutagenesis to obtain various modification variants, the structures of which are shown in Table 1. The parent clones of the clones in Table 1 and Table 2 were both P2-42.

The P2-42 is a VHH antibody the C-terminus of which ends with VTVSS. The clones P2-43 through P2-58 are variants derived from P2-42 by modification of its C-terminus only, with the sequences, other than the amino acid residues undergoing C-terminal modifications (including additions and substitutions) in Table 1, unchanged. The position numbering was performed according to the human germline framework sequence.

The modification variants were tested and validated using the ELISA method in Example 1. The serum samples used were the 8 individual serum samples that tested positive for pre-ADAs in Example 1. The results are shown in FIG. 2 and Table 1. The results in Table 1 show the mean percent signal reduction (%) and the number of positive individuals for each modification variant relative to its parent clone. A greater mean signal reduction (%) indicates that the modification variant has a lower capacity to bind to pre-ADAs, and consequently, a lower likelihood of being neutralized by pre-existing pre-ADAs in the human body.

The calculation formula for the mean signal reduction (%) of the present disclosure was: mean signal reduction% = (arithmetic mean signal value of corresponding sample group - arithmetic mean signal value of P2-42 group)/arithmetic mean signal value of P2-42 group × 100%

The results show that the 6 molecules P2-53, P2-54, P2-55, P2-56, P2-57, and P2-58 exhibited significantly reduced pre-ADA-binding signals.

**Table 1. Reductions in pre-ADA-binding signals of the modification variants**

| No. | Sequence modification (C-terminal sequence) | Mean signal reduction (%) | Number of pre-ADA positive individuals* |
|---|---|---|---|
| P2-42 | Unmodified (VTVSS) | - | 8 |
| P2-43 | C-terminus plus A (VTVSSA) | 43.5% | 4 |
| P2-44 | S113A (VTVSA) | 1.2% | 8 |
| P2-46 | S113F (VTVSF) | -0.9% | 8 |
| P2-47 | S113G (VTVSG) | 3.1% | 8 |
| P2-48 | S113L (VTVSL) | -3.3% | 8 |
| P2-49 | S113P (VTVSP) | 8.0% | 6 |
| P2-50 | S113R (VTVSR) | -4.0% | 8 |
| P2-51 | S113T (VTVST) | 2.5% | 8 |
| P2-52 | S113V (VTVSV) | 4.4% | 8 |
| P2-53 | S113A, C-terminus plus A (VTVSAA) | 38.2% | 4 |
| P2-54 | S112F, S113P (VTVFP) | 49.5% | 2 |
| P2-55 | S112F, S113P, C-terminus plus A (VTVFPA) | 56.7% | 2 |
| P2-56 | S112R, S113P (VTVRP) | 50.7% | 2 |
| P2-57 | S112R (VTVRS) | 48.0% | 3 |
| P2-58 | S 112R, C-terminus plus A (VTVRSA) | 67.9% | 0 |

| | | | |
|---|---|---|---|
| (*The serum samples used in the Table 1 assay were the 8 individual serum samples that tested positive for pre-ADAs in Example 1.) VTVSS, VTVSSA, VTVSF, VTVSG, VTVSL, VTVSR, VTVST, and VTVSV are set forth in SEQ ID NOs: 189-196, respectively. | | | |

### Example 3. pre-ADA Assay of Modification Variants of Single-Domain Antibodies

For the 6 molecules P2-53, P2-54, P2-55, P2-56, P2-57, and P2-58, this example further examined the frequency of pre-ADAs for each modification variant in serum samples taken from 40 healthy donors, to validate the effect of the mutations. The serum samples used were individual serum samples taken from healthy humans (which were the same as the 40 samples in Example 1).

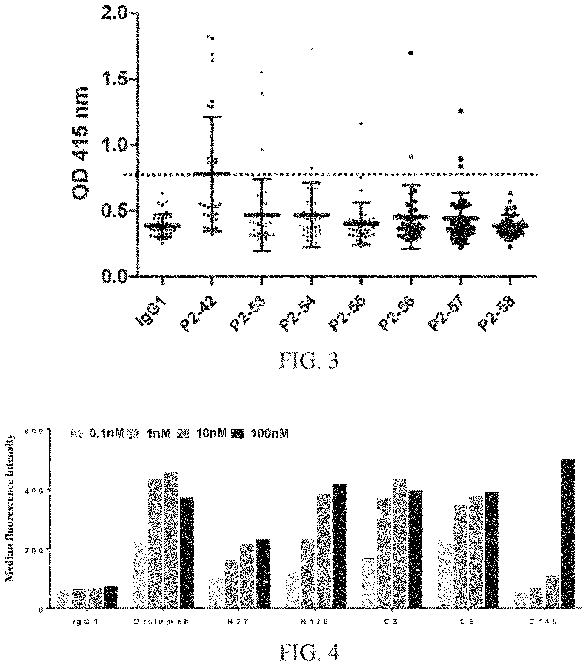

The modification variants were tested and validated using the ELISA method in Example 1. FIG. 3 shows the signal inhibition level of each mutant. Table 2 shows the mean signal inhibition (%) and the frequency of pre-ADA positives for each mutant.

The results show that all clones exhibited a mean signal reduction of close to or over 40%, indicating that the modifications significantly reduced the binding of the single-domain antibodies to pre-ADAs.

**Table 2. Reductions in pre-ADA-binding signals of the modification variants**

| No. | Mean signal reduction (%) | Number of pre-ADA positive individuals* |
|---|---|---|
| P2-42 | - | 21 |
| P2-53 | 41.0% | 6 |
| P2-54 | 39.3% | 6 |
| P2-55 | 49.5% | 3 |
| P2-56 | 41.5% | 6 |
| P2-57 | 42.8% | 5 |
| P2-58 | 51.6% | 1 |

| | | |
|---|---|---|
| (*The serum samples used in the Table 2 assay were the 40 individual serum samples in | | |

### Example 1)

### Example 4. Screening and Preparation for Anti-4-1BB Single-Domain Antibodies

The proteins or polypeptides used in the present disclosure: human 4-1BB protein or polypeptide (Human 4-1BB/TNFSF9 Protein, His Tag, purchased from Acrobiosystems, Cat. No. 41B-H5227), human 4-1BB protein or polypeptide (biotin/His Tag) (Biotinylated Human 4-1BB/TNFRSF9 Protein, Avitag^{™}, His Tag, purchased from Acrobiosystems, Cat. No. 41B-H82E3), and monkey 4-1BB protein or polypeptide (Cynomolgus / Rhesus macaque 4-1BB/TNFRSF9 Protein, His Tag, purchased from Acrobiosystems, Cat. No. 41B-C52H4); the amino acid sequences all start with Leu24 and end with Gln186.

### 1. Alpaca immunization, titer determination, and phage library affinity panning

Alpacas were immunized with a His-tagged human 4-1BB recombinant protein or polypeptide (Acrobiosystems, 41B-H5227) once every two weeks, and a total of four immunizations were performed. In the first immunization, 0.5 mg of antigen and 1 mL of complete Freund's adjuvant (CFA) were mixed well and injected subcutaneously, and in the following three immunizations, 0.25 mg of antigen and 1 mL of incomplete Freund's adjuvant (IFA) were mixed well and injected subcutaneously. Blank serum was collected before immunization, and 50 mL of peripheral blood was collected at one week after the 3rd immunization and one week after the 4th immunization. PBMCs were separated, and total RNA was extracted. The purity was tested, and reverse transcription was performed to obtain DNA. After two rounds of nested PCR, the nanobody target fragment was ligated to a phage display vector. Electroporation was performed to obtain a phage library.

### > Human 4-1BB protein or polypeptide sequence (SEQ ID NO: 5)

LQDPCSNCPAGTFCDNNRNQICSPCPPNSFSSAGGQRTCDICRQCKGVFRTRKEC
SSTSNAECDCTPGFHCLGAGCSMCEQDCKQGQELTKKGCKDCCFGTFNDQKR
GICRPWTNCSLDGKSVLVNGTKERDVVCGPSPADLSPGASSVTPPAPAREPGHSP
Q

In order to obtain an anti-4-1BB nanobody that simultaneously recognizes humans and monkeys, a strategy of two rounds of cross-screening with human and monkey antigens was adopted. The antigens used in the first and second rounds of screening were human 4-1BB and monkey 4-1BB, or monkey 4-1BB and human 4-1BB, respectively. A Gly-HCl acid elution method was adopted in each round of screening, and phages that specifically bind to 4-1BB were eluted. 96 clones (192 clones in total) were randomly selected from the plates of the first and second rounds of titer determination. A phage ELISA was performed to screen for positive clones, and the optical density at 450 nm was measured. The positive clones were sequenced. Based on the sequencing results, sequence alignment and phylogenetic tree analysis were performed, and 14 distinctive sequences, including H27, H170, C3, C5, C145, etc., were selected. The C5 sequence is shown below, and the sequences such as H27, H170, C3, and C145 are not shown.

### > C5 (SEQ ID NO: 6)

QVQLVESGGGLVQPGGSLRLSCSASGFTFSSYAMSWVRQAPGKGLEWVSDINS
GGESTFYEDSVKGRFTISRDNAKNTLYLQMNSLKPEDTAVYYCAKHPLTFTIAT
MNDYDYWGQGTQVTVSS

(Note: The CDRs are underlined)

**Table 3. The CDRs of the anti-4-1BB single-domain antibody C5 (Kabat numbering scheme)**

| Antibody No. | CDR sequences | | Sequence No. |
|---|---|---|---|
| C5 | CDR1 | SYAMS | SEQ ID NO: 7 |
| | CDR2 | DINSGGESTFYEDSVKG | SEQ ID NO: 8 |
| | CDR3 | HPLTFTIATMNDYDY | SEQ ID NO: 9 |

### 2. Expression and purification of VHH-Fc fusion proteins or polypeptides

The sequence of C5 was linked to human IgG1-Fc (SEQ ID NO: 10, mutations are underlined) with the mutations C220A, S267E, and L328F (numbered according to the Eu scheme). The resulting VHH-Fc fusion protein or polypeptide sequence is shown below. Furthermore, mutations such as L234A, L235A, and N297A (according to the Eu numbering scheme) were introduced into the Fc of human IgG1 to completely remove the FcyR-mediated effector function of the antibody (e.g., set forth in SEQ ID NO: 10); S228P (according to the Eu numbering scheme) was introduced into the Fc of human IgG4 to stabilize the antibody molecule, preventing half-molecule formation (e.g., set forth in SEQ ID NO: 11). Both are alternative IgG Fcs. In SEQ ID NOs: 10-12, the Fc mutations are underlined.
> Human IgG1-Fc (containing mutations C220A, S267E, and L328F) (SEQ ID NO: 10) 
EPKSADKTHTCPPCPAPELLGGPSVFLFPPKPKDTLMISRTPEVTCVVVDVEHED
PEVKFNWYVDGVEVHNAKTKPREEQYNSTYRVVSVLTVLHQDWLNGKEYKC
KVSNKAFPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK (SEQ ID NO: 10)
> Human IgG1-Fc (containing mutations C220A, L234A, L235A, and N297A) (SEQ ID NO: 11) 
EPKSADKTHTCPPCPAPEAAGGPSVFLFPPKPKDTLMISRTPEVTCVVVDVSHED
PEVKFNWYVDGVEVHNAKTKPREEQYASTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK
> Human IgG4-Fc (containing mutation S228P) (SEQ ID NO: 12) 
ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGK
> Human IgG1 Fc (SEQ ID NO: 13)

DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNH
YTQKSLSLSPGK

The antibody sequence obtained by linking C5 to SEQ ID NO: 10 is set forth in SEQ ID NO: 14 by way of example.

### > C5-Fc (SEQ ID NO: 14)

QVQLVESGGGLVQPGGSLRLSCSASGFTFSSYAMSWVRQAPGKGLEWVSDINS
GGESTFYEDSVKGRFTISRDNAKNTLYLQMNSLKPEDTAVYYCAKHPLTFTIAT
MNDYDYWGQGTQVTVSSEPKSADKTHTCPPCPAPELLGGPSVFLFPPKPKDTLMI
SRTPEVTCVVVDVEHEDPEVKFNWYVDGVEVHNAKTKPREEQYNSTYRVVSVLTVLH
QDWLNGKEYKCKVSNKAFPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFS
CSVMHEALHNHYTQKSLSLSPGA

(Note: The Fc is italicized)

Plasmids were constructed and transiently transfected into cells, and antibodies were expressed and purified. Analysis showed that the antibodies of interest were obtained.

### Example 5. Assay for Antigen-Binding Activity and Agonist Activity of Anti-4-1BB Single-Domain Antibodies

### 1. Assay for capacity to bind to 4-1BB antigen

The activity of the anti-4-1BB single-domain antibodies in binding to human 4-1BB protein or polypeptide was measured by flow cytometry.

HEK293-Hu4-1BB cells were obtained by transient transfection of HEK293 cells (ATCC CRL-1573) with a gene expressing human 4-1BB protein or polypeptide (CD137 cDNA ORF Clone, Human, C-OFPSpark tag; purchased from Sino Biological, Cat#HG10041-ACR). The cell culture medium was DMEM (Gibco, Cat#11995065) containing 10% fetal bovine serum. The experimental medium was sterile PBS (phosphate-buffered saline, pH 7.40) containing 2% fetal bovine serum. HEK293-Hu4-1BB cells were washed with the experimental medium twice and seeded into a 96-well U-bottom plate at 1 × 10⁵ HEK293-Hu4-1BB cells per well. Different concentrations of the anti-4-1BB single-domain antibody VHH-Fc samples to be tested were added. The cells were incubated at 4 °C for 1 h and then washed with the experimental medium twice. Then the goat anti-human IgG (H+L) Alexa Fluor 488 antibody (Thermo, Cat#A11013) was added. After two washes, fluorescence signal values were measured using a flow cytometer. Urelumab was used as a positive control. The MFI values for the antibodies are shown in FIG. 4.

The results show that the involved anti-4-1BB single-domain antibodies had different capacities to bind to 4-1BB on the surface of HEK293-Hu4-1BB cells, and that C5 had good cell membrane surface antigen-binding activity.

### 2. Assay for agonist activity on 4-1BB signaling pathway

The agonist activity of the anti-4-1BB single-domain antibodies was assessed using a 4-1BB/NF-κB reporter gene.

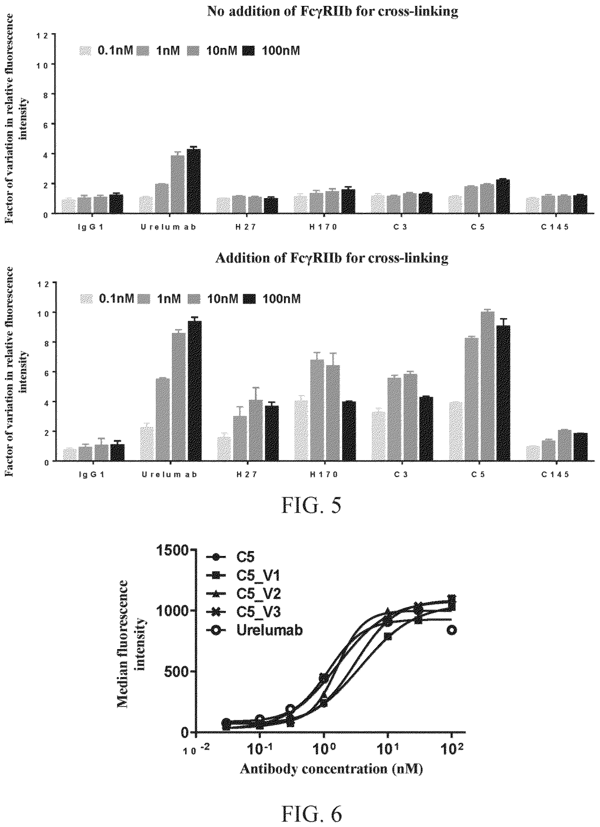

HEK293-Hu4-1BB/NF-κB double transfection cells were obtained by transient transfection of HEK293 cells (ATCC CRL-1573) with a gene expressing human 4-1BB (CD137 cDNA ORF Clone, Human, C-OFPSpark tag; purchased from Sino Biological, Cat#HG10041-ACR) and the NF-κB reporter gene (pGL4.32[luc2P/NF-κB-RE/Hygro] Vector, purchased from Promega, Cat# E849A). 4-1BB activation can be characterized by the level of activation of the NF-κB signaling pathway. HEK293 cells highly expressing FcyRIIb were obtained by transient transfection of HEK293 cells with FcyRIIb plasmids (CD32B/Fcgr2b cDNA ORF Clone, Human, N-His tag; purchased from Sino Biological, Cat#HG10259-NH). The cell culture medium was DMEM (Gibco, Cat#11995065) containing 10% fetal bovine serum. HEK293-Hu4-1BB/NF-κB cells (2 × 10⁶/mL) were plated onto a 96-well cell culture plate at 50 µL; 40 µL of medium or FcyRIIb-expressing HEK293 cells (2.5 × 10⁶/mL) was added; and 10× serially diluted anti-4-1BB single-domain antibodies to be tested were added at 10 µL/well. The plate was incubated at 37 °C for 6 h. The cells were taken out. An equal volume of Bio-Glo Luciferase Assay System reagent (Promega, Cat# G7940) was added to each well, and the plate was incubated for 5 min in the dark. Fluorescence signals were measured using an Envision microplate reader (PerkinElmer, 2150). EC₅₀ values and Emax values (relative to the fluorescence intensity of the antibody-free group) were calculated, and the EC₅₀ values were used to evaluate the agonist activity of the anti-4-1BB single-domain antibodies on *in vitro* cells. The results are shown in FIG. 5 and Table 4.

The results show that when no FcyRIIb (i.e., CD32b) was added for positive cross-linking, the involved anti-4-1BB single-domain antibodies exhibited much weaker activation of the 4-1BB/NF-κB luciferase reporter gene signaling pathway than the control urelumab, indicating that the anti-4-1BB single-domain antibodies of the present disclosure are safer; after FcyRIIb was added for cross-linking, the involved anti-4-1BB single-domain antibodies exhibited significant activation of the 4-1BB/NF-κB luciferase reporter gene signaling pathway, with C5 having the greatest activating capacity, which was comparable to that of the control urelumab. Based on these activity results, the sequence C5, which had low background activation before FcyRIIb cross-linking and stronger activation activity after FcyRIIb cross-linking, was selected and humanized.

**Table 4. The EC₅₀ values for the agonistic activity of the anti-4-1BB single-domain antibodies on the 4-1BB/NF-κB luciferase reporter gene**

| No. | FcyRIIb cross-linking | | No FcyRIIb cross-linking | |
|---|---|---|---|---|
| | Emax | EC₅₀ (uM) | Emax | EC₅₀ (nM) |
| IgG | 1.12 | - | 1.10 | - |
| Urelumab | 9.39 | 0.027 | 4.30 | 2.185 |
| C5 | 10.04 | ~ 0.8144 | 2.26 | - |

| | | | | |
|---|---|---|---|---|
| (Note: "-" indicates that the measurement was beyond the limit of detection) | | | | |

### Example 6. Engineering of Anti-4-1BB Single-Domain Antibodies

### 1. Humanization engineering

The amino acids of the CDRs and FRs (human framework regions) of the C5 sequence were numbered using the Kabat numbering scheme. The FR1+CDR1, FR2+CDR2, and FR3+CDR3 sequences were matched with an antibody germline database to obtain FR human germline templates with relatively high homology. FR1 was derived from IGHV3-64*04, FR2 from IGHV3-23*03, and FR3 from IGHV3-74*01. The above human germline FRs were inserted into the original sequences as replacements to reduce the immunogenicity produced in the human body. The key amino acids that affect the structures and functions of the antibodies were back-mutated to restore binding affinity and activity. The humanized sequences were as follows.
>C5_V1 (SEQ ID NO: 15) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCARHPLTFTIAT
MNDYDYWGQGTLVTVSS
> C5_V2 (SEQ ID NO: 16) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTFTIAT
MNDYDYWGQGTLVTVSS
> C5_V3 (SEQ ID NO: 17) 
QVQLVESGGGLVQPGGSLRLSCSASGFTFSSYAMSWVRQAPGKGLEWVSDINS
GGESTFYEDSVKGRFTISRDNAKNTLYLQMNSLKPEDTAVYYCAKHPLTFTIAT
MNDYDYWGQGTLVTVSS
 (Note: The CDRs are underlined)

The above 3 humanized sequences were each linked to human IgG1-Fc (SEQ ID NO: 10). Plasmids were constructed. Transient transfection, expression, and purification were performed. The specific procedure was as follows: 60 µL of transfection reagent was diluted with culture solution and then mixed with 15 µg of plasmids. After 15 min of incubation at 37 °C, the mixed transfection solution was added dropwise to 30 mL of cell solution. After one week of culture and expression on a shaker, the supernatant was collected. It was then subjected to ProteinA affinity purification, with a citrate buffer (pH 3.4) as the eluent. Finally, the eluate was dialyzed against 1× PBS buffer and cryopreserved.

### 2. Removal of TCE sites

Subsequently, we performed T-cell Epitope (TCE) prediction on the C5_V2 sequence and modified the CDR3 sequence of C5_V2 based on the prediction results to reduce the number of TCEs. Mutation of F to Y (F99Y, according to the Kabat numbering scheme) in the CDR3 region yielded a TCE-optimized version of the C5_V2 sequence, which was designated C5_V2-YTI, and the sequence of which was as follows:

### > C5_V2-YTI (SEQ ID NO: 18)

QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSS

That is, according to the Kabat numbering scheme, the amino acid sequence of the CDR1 in C5_V2-YTI is set forth in SEQ ID NO: 7, the amino acid sequence of the CDR2 is set forth in SEQ ID NO: 8, and the amino acid sequence of the CDR3 is HPLTYTIATMNDYDY (SEQ ID NO: 19).

The sequences of the above C5_V2-YTI, C5, C5_V1, C5_V2, and C5_V3 were linked to human IgG1-Fc (SEQ ID NO: 10). Plasmids were constructed. Transient transfection, expression, and purification were performed. The resulting products were used for subsequent assays.

### Example 7. Assay for Antigen-Binding Activity and Agonist Activity of Engineered Anti-4-1BB Single-Domain Antibodies

### 1. Assay for capacity to bind to 4-1BB antigen

The antigen-binding activity of the humanized antibodies was measured using a FACS assay as described in Example 2. The results are shown in FIG. 6 and Table 5.

The results show that the involved humanized anti-4-1BB single-domain antibodies had different capacities to bind to 4-1BB on the surface of HEK293-Hu4-1BB cells, and that C5_V1, C5_V2, and C5_V3 all retained good binding activity.

**Table 5. The EC₅₀ values for the affinity of the pre-/post-humanization anti-4-1BB single-domain antibodies for a cell strain highly expressing human 4-1BB**

| Antibody No. | EC₅₀ (nM) | Maximal fluorescence value |
|---|---|---|
| C5 | 3.196 | 1101 |
| C5_V1 | 3.789 | 1036 |
| C5_V2 | 1.624 | 1031 |
| C5_V3 | 1.651 | 1096 |
| Urelumab | 1.184 | 1005 |

### 2. Assay for agonist activity on 4-1BB signaling pathway

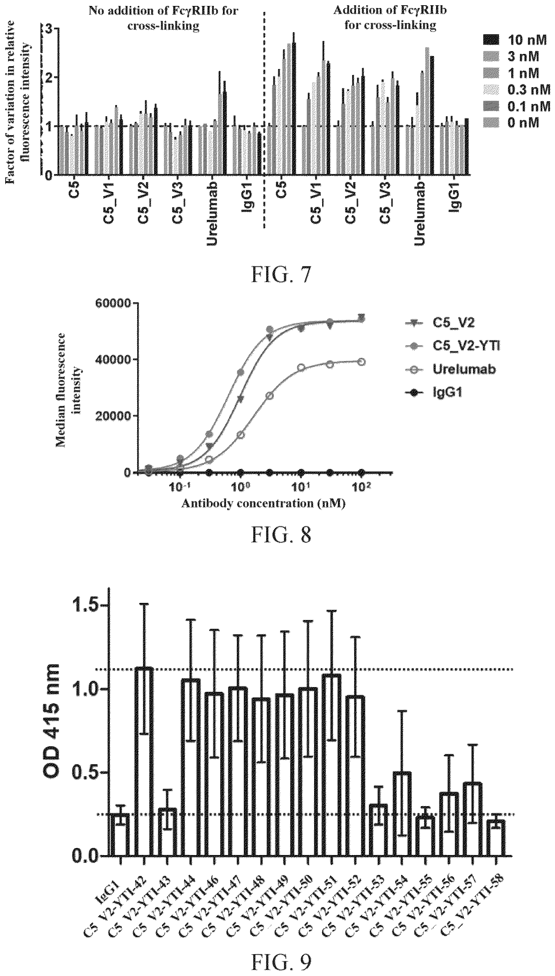

The activation of 4-1BB/NF-xB signaling by the humanized antibodies was measured using an NF-κB luciferase reporter gene assay as described in Example 2. The results are shown in FIG. 7 and Table 6.

The results show that after the addition of FcyRIIb for cross-linking, the involved humanized anti-4-1BB single-domain antibodies exhibited activation of the 4-1BB/NF-κB luciferase reporter gene signaling pathway, with the activating capacities of C5, C5_V1, C5_V2, and C5_V3 being comparable to that of the urelumab (from WO2005035584A) control.

**Table 6. The activation of the NF-κB signaling pathway by the pre-/post-humanization anti-4-1BB single-domain antibodies**

| Antibody No. | Activation of HEK293-4-1BB cells | | FcyRIIb-mediated activation of HEK293-4-1BB cells | |
|---|---|---|---|---|
| | Activity | Factor of variation in Emax | Activity | Factor of variation in Emax |
| C5 | - | 1.08 | +++ | 2.71 |
| C5_V1 | - | 1.14 | ++ | 2.28 |
| C5_V2 | - | 1.37 | ++ | 2.02 |
| C5_V3 | - | 1.01 | + | 1.83 |
| Urelumab | + | 1.70 | +++ | 2.61 |

| | | | | |
|---|---|---|---|---|
| (Note: For a factor of variation in Emax of <1.5, the activity is defined as "-"; for 1.5-2, as "+"; for 2-2.5, as "++"; for 2.5-3, as "+++"; and for >3, as "++++") | | | | |

A comparison of the antigen-binding activity of C5_V2 and C5_V2-YTI was made using a FACS assay as described in Example 5. The results are shown in FIG. 8 and Table 7. The results show that the involved C5_V2-YTI retained good binding activity to the 4-1BB on the surface of HEK293-Hu4-1BB cells, which is comparable to that of C5_V2.

**Table 7. The EC₅₀ values for the affinity of C5_V2 and C5_V2-YTI for a cell strain highly expressing human 4-1BB**

| Antibody No. | EC₅₀ (nM) | Maximal fluorescence value |
|---|---|---|
| C5_V2 | 0.98 | 53669 |
| C5_V2-YTI | 0.62 | 53825 |
| Urelumab | 1.64 | 39666 |
| IgG1 | - | 67 |

### Example 8. Amino Acid Engineering of VH Frameworks (FRs) of Anti-4-1BB Single-Domain Antibody and pre-ADA Assay

The anti-4-1BB single-domain antibody of the present disclosure, C5 V2-YTI, was subjected to C-terminal engineering according to Table 8.

**Table 8. Mutants derived from C5_V2-YTI by C-terminal engineering**

| No. | Sequence modification (C-terminal sequence) |
|---|---|
| C5_V2-YTI-42 | Unmodified (VTVSS) |
| C5_V2-YTI-43 | C-terminus plus A (VTVSSA) |
| C5_V2-YTI-44 | S113A (VTVSA) |
| C5_V2-YTI-46 | S113F (VTVSF) |
| C5_V2-YTI-47 | S113G (VTVSG) |
| C5_V2-YTI-48 | S113L (VTVSL) |
| C5_V2-YTI-49 | S113P (VTVSP) |
| C5_V2-YTI-50 | S113R (VTVSR) |
| C5_V2-YTI-51 | S113T (VTVST) |
| C5_V2-YTI-52 | S113V (VTVSV) |
| C5_V2-YTI-53 | S113A, C-terminus plus A (VTVSAA) |
| C5_V2-YTI-54 | S112F, S113P (VTVFP) |
| C5_V2-YTI-55 | S112F, S113P, C-terminus plus A (VTVFPA) |
| C5_V2-YTI-56 | S112R, S113P (VTVRP) |
| C5_V2-YTI-57 | S112R (VTVRS) |
| C5_V2-YTI-58 | S112R, C-terminus plus A (VTVRSA) |

> C5_V2-YTI-42 (SEQ ID NO: 20) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSS
> C5_V2-YTI-43 (SEQ ID NO: 21) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSSA
> C5_V2-YTI-44 (SEQ ID NO: 22) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSA
> C5_V2-YTI-46 (SEQ ID NO: 23) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSF
> C5_V2-YTI-47 (SEQ ID NO: 24) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSG
> C5_V2-YTI-48 (SEQ ID NO: 25) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSL
> C5_V2-YTI-49 (SEQ ID NO: 26) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSP
> C5_V2-YTI-50 (SEQ ID NO: 27) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSR
> C5_V2-YTI-51 (SEQ ID NO: 28) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVST
> C5_V2-YTI-52 (SEQ ID NO: 29) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSV
> C5_V2-YTI-53 (SEQ ID NO: 30) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVSAA
> C5_V2-YTI-54 (SEQ ID NO: 31) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVFP
> C5_V2-YTI-55 (SEQ ID NO: 32) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVFPA
> C5_V2-YTI-56 (SEQ ID NO: 33) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVRP
> C5_V2-YTI-57 (SEQ ID NO: 34) 
QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVRS
> C5_V2-YTI-58 (SEQ ID NO: 35)

QVQLVESGGGLVQPGGSLRLSCSASGFTFSSYAMSWVRQAPGKGLEWVSDINS
GGESTFYEDSVKGRFTISRDNAKNTLYLQMNSLRAEDTAVYYCAKHPLTYTIAT
MNDYDYWGQGTLVTVRSA

The N-termini of the sequences set forth in SEQ ID NOs: 20-35 were linked to the C-terminus of IgG1 Fc (SEQ ID NO: 13) by a linker of GGGG (SEQ ID NO: 197), and the Table 9 and Table 10 assays were performed. Exemplary sequences are shown below, where IgG1 Fc is italicized and the linker is underlined.

### > C5_V2-YTI-42 (SEQ ID NO: 36)

DKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPGKG
GGGQVQLVESGGGLVQPGGSLRLSCSASGFTFSSYAMSWVRQAPGKGLEWVSD
INSGGESTFYEDSVKGRFTISRDNAKNTLYLQMNSLRAEDTAVYYCAKHPLTYTI
ATMNDYDYWGQGTLVTVSS

The mutants derived from C5_V2-YTI by C-terminal engineering were subjected to an ELISA according to the experimental method of Example 2. The results are shown in Table 9. The results show that the 6 molecules C5_V2-YTI-53, C5_V2-YTI-54, C5_V2-YTI-55, C5_V2-YTI-56, C5_V2-YTI-57, and C5_V2-YTI-58 exhibited significantly reduced pre-ADA-binding signals.

**Table 9. Reductions in pre-ADA-binding signals of the modification variants derived from C5_V2-YTI by C-terminal engineering**

| No. | Sequence modification (C-terminal sequence) | Mean signal reduction (%) | Number of pre-ADA positive individuals* |
|---|---|---|---|
| C5_V2-YTI-42 | Unmodified (VTVSS) | - | 8 |
| C5_V2-YTI-43 | C-terminus plus A (VTVSSA) | 75.1% | 1 |
| C5_V2-YTI-44 | S113A (VTVSA) | 6.2% | 7 |
| C5_V2-YTI-46 | S113F (VTVSF) | 13.4% | 7 |
| C5_V2-YTI-53 | S113A, C-terminus plus A (VTVSAA) | 73.1% | 2 |
| C5_V2-YTI-54 | S112F, S113P (VTVFP) | 55.7% | 4 |
| C5_V2-YTI-55 | S112F, S113P, C-terminus plus A (VTVFPA) | 79.5% | 0 |
| C5_V2-YTI-56 | S112R, S113P (VTVRP) | 66.6% | 4 |
| C5_V2-YTI-57 | S112R (VTVRS) | 61.4% | 4 |
| C5_V2-YTI-58 | S 112R, C-terminus plus A (VTVRSA) | 81.4% | 0 |

| | | | |
|---|---|---|---|
| (*The serum samples used in the Table 9 assay were the 8 individual serum samples that tested positive for pre-ADAs in Example 1) | | | |

The mutants derived from C5_V2-YTI by C-terminal engineering were subjected to an ELISA according to the experimental method of Example 3. The results are shown in Table 10. The results show that all clones exhibited a mean signal reduction of close to or over 40%, indicating that the modifications significantly reduced the binding of the single-domain antibodies to pre-ADAs.

**Table 10. Reductions in pre-ADA-binding signals of the modification variants derived from C5_V2-YTI by C-terminal engineering**

| No. | Mean signal reduction (%) | Number of pre-ADA positive individuals* |
|---|---|---|
| C5_V2-YTI-42 | - | 21 |
| C5_V2-YTI-53 | 50.8% | 7 |
| C5_V2-YTI-54 | 40.9% | 7 |
| C5_V2-YTI-55 | 57.0% | 3 |
| C5_V2-YTI-56 | 46.5% | 7 |
| C5_V2-YTI-57 | 45.2% | 8 |
| C5_V2-YTI-58 | 59.5% | 2 |

| | | |
|---|---|---|
| (*The serum samples used in the Table 10 assay were the 40 individual serum samples in Example 1) | | |

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A modified immunoglobulin single variable domain, comprising:
a C-terminus the amino acid sequence of which is represented by X₁X₂(X₃)ₙ or VTVX₁X₂(X₃)ₙ, wherein X₁ is selected from the group consisting of S, F, and R, X₂ is selected from the group consisting of A, P, and S, and X₃ is A or absent; n is an integer of 0-5;
when n is 0, X₃ is absent;
when n is 1-5, X₃ is A;
preferably, the modified immunoglobulin single variable domain comprises a C-terminus consisting of any one of the following (a)-(e):
(a) SAA or VTVSAA;
(b) FP or VTVFP;
(c) FPA or VTVFPA;
(d) RS or VTVRS; and
(e) RSA or VTVRSA.

2. A modified immunoglobulin single variable domain, comprising a heavy chain framework sequence derived from a human germline, and having an amino acid modification of 112F or 112R at position 112 relative to the human germline heavy chain framework sequence, wherein
optionally, the immunoglobulin single variable domain further has an amino acid modification of 113P at position 113 relative to the human germline heavy chain framework sequence, or has an S at position 113;
preferably, the immunoglobulin single variable domain has an amino acid residue combination of any one of the following (f)-(h) at position 112 and position 113 relative to the human germline framework sequence:
(f) 112F and 113P;
(g) 112R and 113S; and
(h) 112F and 113S.

3. A modified immunoglobulin single variable domain, comprising a heavy chain framework sequence derived from a human germline, having an S at position 112 relative to the human germline heavy chain framework sequence, having an amino acid modification of 113A or 113P at position 113 relative to the human germline heavy chain framework sequence, and also having an amino acid extension of 1 to 5 amino acid residues after position 113, wherein the amino acid extension is preferably A, AA, or AAA.

4. The modified immunoglobulin single variable domain according to any one of the preceding claims, being selected from the group consisting of a VH single-domain antibody or a nanobody, wherein the VH single-domain antibody is preferably of human origin, and the nanobody is preferably of camelid origin or humanized.

5. The modified immunoglobulin single variable domain according to any one of the preceding claims, wherein the modified immunoglobulin single variable domain has reduced binding to pre-anti-drug antibodies (pre-ADAs) or anti-drug antibodies (ADAs), as compared to an unmodified immunoglobulin single variable domain having a C-terminus the amino acid sequence of which is VTVSS (SEQ ID NO: 189).

6. The modified immunoglobulin single variable domain according to any one of the preceding claims, specifically binding to a target antigen and comprising:
a) three complementarity-determining regions (CDRs) specific for the target antigen, and
b) four framework regions (FRs).

7. The modified immunoglobulin single variable domain according to any one of the preceding claims, comprising:
a) one or more amino acid modifications at position 14, position 41, and position 108 relative to the human germline heavy chain framework sequence, preferably 14A, 14K, 14Q, or 14T; and/or 41A; and/or 108A or 108Q; and/or
b) an amino acid modification at position 11 and/or position 88 relative to the human germline heavy chain framework sequence, preferably 11K, 11R, 11D, or 11E; and/or 88E, 88D, 88R, or 88K.

8. The modified immunoglobulin single variable domain according to any one of the preceding claims, wherein the immunoglobulin single variable domain specifically binds to 4-1BB;
preferably, the immunoglobulin single variable domain comprises a CDR1, a CDR2, and a CDR3 from any one of the amino acid sequences of SEQ ID NOs: 6 and 15-18, and the CDR1, CDR2, and CDR3 are defined according to the Kabat, IMGT, Chothia, AbM, or Contact numbering scheme;
more preferably, the immunoglobulin single variable domain comprises a CDR1, a CDR2, and a CDR3 set forth in SEQ ID NOs: 7, 8, and 9, respectively, or a CDR1, a CDR2, and a CDR3 set forth in SEQ ID NOs: 7, 8, and 19, respectively;
most preferably, the amino acid sequence of the immunoglobulin single variable domain that specifically binds to 4-1BB is set forth in any one of SEQ ID NOs: 21 and 30-35 or having at least 90%, optionally at least 95%, identity thereto.

9. A protein or polypeptide, comprising at the C-terminus the modified immunoglobulin single variable domain according to any one of claims 1-8.

10. The protein or polypeptide according to claim 9, being an antibody, preferably a monovalent, bivalent, or multivalent, monospecific, bispecific, or multispecific antibody.

11. The protein or polypeptide according to claim 9 or 10, targeting at least two different antigens, or at least two different domains or epitopes of the same antigen, wherein preferably, at least one of the at least two antigens or the same antigen is 4-1BB.

12. The protein or polypeptide according to any one of claims 9-11, also comprising an immunoglobulin Fc region, preferably the Fc region of human IgG1, human IgG2, human IgG3, or human IgG4.

13. The protein or polypeptide according to claim 12, wherein the N-terminus of the modified immunoglobulin single variable domain is linked to the C-terminus of the Fc region directly or by a linker.

14. The protein or polypeptide according to any one of claims 9-13, being a therapeutic or prophylactic antibody.

15. A polynucleotide, encoding the modified immunoglobulin single variable domain according to any one of claims 1-8 or the protein or polypeptide according to any one of claims 9-14.

16. A vector, comprising or expressing the polynucleotide according to claim 15.

17. A pharmaceutical composition, comprising the modified immunoglobulin single variable domain according to any one of claims 1-8, the protein or polypeptide according to any one of claims 9-14, the polynucleotide according to claim 15, or the vector according to claim 16, and at least one pharmaceutically acceptable excipient, diluent, or carrier.

18. Use of the modified immunoglobulin single variable domain according to any one of claims 1-8 or the protein or polypeptide according to any one of claims 9-14 for the preparation of a medicament having reduced binding to pre-ADAs or ADAs in a subject.

19. A method for preventing or treating a disease, comprising administering to a subject in need thereof a prophylactically or therapeutically effective amount of the modified immunoglobulin single variable domain according to any one of claims 1-8 or the protein or polypeptide according to any one of claims 9-14, wherein preferably, the disease is a cell proliferative disease, a metabolic disease, an autoimmune disease, or an infectious disease; more preferably, the cell proliferative disease is a cancer.

20. A method for reducing binding of an immunoglobulin single variable domain to pre-ADAs or ADAs, wherein the method comprises: allowing the C-terminus of the immunoglobulin single variable domain to comprise a C-terminus the amino acid sequence of which is represented by X₁X₂(X₃)ₙ or VTVX₁X₂(X₃)ₙ, wherein Xi is selected from the group consisting of S, F, and R, X₂ is selected from the group consisting of A, P, and S, and X₃ is A or absent; n is an integer of 0-5;
when n is 0, X₃ is absent;
when n is 1-5, X₃ is A;
preferably, the modified immunoglobulin single variable domain comprises a C-terminus consisting of any one of the following (a)-(e):
(a) SAA or VTVSAA;
(b) FP or VTVFP;
(c) FPA or VTVFPA;
(d) RS or VTVRS; and
(e) RSA or VTVRSA.
